# EUROPEAN PATENT APPLICATION

(11) **EP 1 801 212 A1**
(43) Date of publication of application: **27.06.2007**
(21) Application number: 05077951.1
(22) Date of filing: 21.12.2005
(51) Int. Cl.: C12N 15/10, C12Q 1/68, C40B 30/06, C40B 30/08, C40B 40/08

(54) **Selection of biocatalysts for chemical synthesis**

(71) Applicant: Eidgenössische Technische Hochschule Zürich, 8093 Zürich (CH)
(72) Inventor: Witholt, Bernard, 8032 Zürich (CH); van Beilen, Jan Berthold, 8049 Zürich (CH); van Sint Fiet, Stephan Johannes Hubertus, 8046 Zürich (CH)
(74) Representative: Winckels, Johannes Hubertus F.

(57) **Abstract**

The present invention relates to a method of detecting among a population of candidate biocatalysts a biocatalyst capable of catalyzing a chemical conversion reaction from a substrate to a product, said method comprising the steps of:
a) providing a host cell comprising:
- at least one product-inducible expression system comprising nucleic acid encoding at least one detector gene operably linked to a regulatory element, wherein the expression of said detector gene is inducible by said product, and
- at least one biocatalyst expression system comprising nucleic acid encoding at least one candidate biocatalyst, wherein said at least one candidate biocatalyst is selected from said population of candidate biocatalysts,

b) contacting said host cell with said substrate under conditions wherein said nucleic acid encoding at least one candidate biocatalyst is expressed in said host cell and wherein said substrate is allowed to contact said candidate biocatalyst, and wherein said substrate is converted into said product in case said candidate biocatalyst is capable of catalyzing said reaction, and
c) detecting said host cell as one comprising a biocatalyst capable of catalyzing said chemical conversion reaction on the basis of the expression of said detector gene.

## Description

### FIELD OF THE INVENTION

The invention relates to production of (bio)chemicals and more in particular to systems for the detection of biocatalysts capable of catalyzing the reaction from a substrate to a desired (bio)chemical product.

### BACKGROUND OF THE INVENTION

Biocatalyst technology, as a part of chemical biotechnology, is increasingly important as a tool for chemical synthesis. Its application is driven by consumer demand for new products, by industrial attempts at reducing costs and increase profits, and by government regulatory pressures. Current applications of biocatalysts include the production of high fructose corn syrup, aspartame, semi-synthetic penicillins and novel cancer drugs. In fact it is expected that biocatalysis will revolutionize industrial chemistry in the next few decades, producing compounds from specialties to bulk chemicals. This is so because the potential pool of native and modified biocatalysts is enormous.

Biocatalytic enzymes play a pivotal role in organic synthesis processes. An increasing number of industrial products, ranging from pharmaceutical intermediates to bulk chemicals, are manufactured with processes that involve one or more biocatalytic steps, foreshadowing the emergence of a significant biotechnology based chemical industry. A key factor in this development is the availability of biocatalysts that enable good overall process efficiency and low production costs. This requires enzymes that show the necessary regio- and enantioselectivity, activity, and stability for desired bioconversions, under practical process conditions.

Much effort is devoted to finding or developing enzymes that meet these criteria, both in academia, where research centers focusing on biocatalysis have lately become popular, and in industry, where growing numbers of young biotech and established pharmaceutical and chemical companies are now developing new biocatalysts.

A much used approach to finding new biocatalysts is based on screening processes. In principle, there is an immense biocatalytic diversity available in the biosphere, and this natural diversity can be synthetically extended in the laboratory using for instance DNA shuffling, directed evolution or site directed mutagenesis techniques. Strategies for enzyme discovery and optimization are generally based on screening these resources. To find desired biocatalysts in this pool by slow and costly screening techniques limits the development of new biocatalysts.

The usual approach in developing a new biocatalyst is to start with one or several known enzymes or corresponding DNA sequences, or if these are not available, to screen for desired activities among enzyme or expressed DNA libraries, in strain collections or in environmental samples containing native strains and DNA (see Burton *et al.,* 2002; Gabor *et al.,* 2004). Once promising enzymes are identified, these can then be improved by design or with various random mutagenesis techniques, in attempts to meet desired functional criteria (see Kim *et al.,* 2003; Sutherland, 2000). This combination of an immense natural diversity from which enzymes can be recruited, and an impressive tool set to further improve native enzymes, suggests that there are few conceptual limits to the enzyme structures that might be found or generated.

There are however practical limits to the testing of new enzyme activities, due to the difficulty of screening for desired biocatalysts among the immense numbers of enzymes generated by the approaches described above (see Burton *et al.,* 2002; Gabor *et al.,* 2004; Sutherland, 2000). In typical screening experiments, natural isolates or hosts containing suitable enzyme banks are grown on solid media or in micro-cultures. Various assay methods are then used to screen for desired activities, including the use of chromogenic or fluorogenic substrates (see Goddard and Reymond, 2004a, 2004b), which enable high throughput screening but generally detect analogs rather than specific products. It is also possible to analyze and quantitate products with analytical techniques such as gas chromatography, high-performance liquid chromatography, mass spectrometry, or NMR (see Reetz, 2001, 2004), but these methods are expensive and generally slow (see Reetz, 2004). Thus, it is difficult to combine direct screening for desired biocatalytic activities with high throughput, and this limits the size of potential enzyme libraries that can be tested in practice.

In fact, it is an aim of the present invention to provide a system for finding biocatalysts which is fast and cost-effective and that can be used in a high throughput mode.

Furthermore, it is an aim of the present invention to provide systems for finding biocatalysts for specific products as well as for chemical analogs of desired products.

### SUMMARY OF THE INVENTION

The present invention now relates to the use of an inducible expression system controlled by the presence in a host cell of a specific product the presence of which is dependent on the presence of a functional enzyme capable of catalyzing the reaction from at least one (generally extracellularly added) substrate to a desired specific product.

In a first aspect, the present invention relates to a method of detecting among a population of candidate biocatalysts a biocatalyst capable of catalyzing a chemical conversion reaction from a substrate to a product, said method comprising the steps of:
a) providing a host cell comprising:
   - at least one product-inducible expression system comprising nucleic acid encoding at least one detector gene operably linked to a regulatory element, wherein the expression of said detector gene is inducible by said product, and
   - at least one biocatalyst expression system comprising nucleic acid encoding at least one candidate biocatalyst, wherein said at least one candidate biocatalyst is selected from said population of candidate biocatalysts;
b) contacting said host cell with said substrate under conditions wherein said nucleic acid encoding at least one candidate biocatalyst is expressed in said host cell and wherein said substrate is allowed to contact said candidate biocatalyst, and wherein said substrate is converted into said product in case said candidate biocatalyst is capable of catalyzing said reaction, and
c) detecting said host cell as one comprising a biocatalyst capable of catalyzing said chemical conversion reaction on the basis of the expression of said detector gene.

The present invention pertains to various embodiments of this aspect. The product-inducible expression system and biocatalyst expression systems may be introduced into a host cell separately or simultaneously, or one system may be native for the host cell. In general, it will be easier to introduce the product-inducible expression system into a suitable host strain, thereby producing a detector strain. Next, libraries of biocatalyst expression systems may then be introduced into the detector strain. Alternatively, a product-inducible expression system may be introduced into a population of similar organisms each of which contain one or more dissimilar genes that encode a biocatalyst that potentially has the desired bioconversion properties. The referred population of organisms is herein said to comprise or represent a library of candidate biocatalysts. The library may consist of variants of a gene which encodes a known or suspected desired biocatalyst. Variants of a gene may for instance be produced in the laboratory with one of several known techniques, such as site directed mutagenesis, directed evolution or DNA shuffling techniques. Alternatively, the dissimilar genes may constitute cloned nucleic acid fragments derived from one source organism (e.g. a shotgun cloned genome) or derived from many source organisms (e.g. a metagenome). Alternatively the library may consist of different known or unknown organisms, each of which naturally comprises one or more candidate biocatalysts and which organisms are modified by the introduction of the product-inducible expression system.

Thus, in one preferred embodiment, the invention relates to a method wherein step a) comprises the provision of a multitude of host cells that represents said population of candidate biocatalysts, wherein said multitude of host cells:
- is a library of cells of a single cell type wherein essentially each host cell comprises a different cloned nucleic acid fragment encoding at least one candidate biocatalyst, or
- are cells of different cell types wherein essentially each host cell comprises a different candidate biocatalyst.

In another preferred embodiment of a method of the invention, the condition under which said host cell is contacted with said substrate is such that it provides an inducer for activating the expression of said at least one biocatalyst expression system.

In another preferred embodiment, the step of detecting said host cell as a cell comprising a biocatalyst capable of catalyzing said chemical conversion reaction, preferably comprises the survival of such cells under selective growth conditions. This means that the detection of cells that contain active biocatalysts is preferably based on the expression of a detector gene that enables the cells to survive under conditions that are lethal or growth inhibiting for cells that do not express the detector gene. Such conditions may for instance comprise a growth medium comprising an antibiotic compound for which the resistance is encoded by said detector gene.

The detection method allows the selection of at least one candidate biocatalyst from said population of candidate biocatalysts if the required product capable of inducing detector gene transcription is produced from said substrate. The detection method may therefore additionally comprise the selection of a biocatalyst, which is essentially performed by selection of the host cell. It is therefore envisioned that the detection method of the present invention may comprise an additional step d) wherein said biocatalyst is provided by selecting and providing the host cell(s) detected in step c).

In another aspect, the present invention relates to a host cell suitable for use in a method of the invention as described above, said host cell comprising:
a) at least one product-inducible expression system comprising nucleic acid encoding at least one detector gene operably linked to a regulatory element, wherein the expression of said detector gene is inducible by said product, and
b) at least one biocatalyst expression system comprising nucleic acid encoding at least one candidate biocatalyst, wherein said at least one candidate biocatalyst is selected from said population of candidate biocatalysts.

In a preferred embodiment, the host cell is one cell out of multitude of host cells that represents said population of candidate biocatalysts, wherein said multitude of host cells:
- is a library of cells of a single cell type wherein essentially each host cell comprises a different cloned nucleic acid fragment encoding at least one candidate biocatalyst, or
- are cells of different cell types wherein essentially each host cell comprises a different candidate biocatalyst.

In another aspect, the present invention provides a method of selecting among a population of candidate substrates a substrate capable of being converted into a product in a biocatalyst-catalyzed chemical conversion reaction, said method comprising the steps of:
a) providing a host cell according to the present invention;
b) subjecting said host cell to conditions wherein said detector gene allows for the detection of cells capable of forming said product, and
c) selecting said substrate on the basis of the expression of said detector gene.

In yet another aspect, the present invention provides a method of producing a (bio)chemical compound wherein said compound is the product of a biocatalyst-catalyzed chemical conversion reaction for converting substrate into product, said method comprising the steps of:
a) detecting and providing a biocatalyst by a method of the present invention;
b) producing said biocatalyst by one of several possible transgenic methods in a production strain, or using the selected host cell as a production strain;
c) providing a reaction mixture comprising said production strain and a substrate for the chemical conversion reaction catalyzed by said biocatalyst, and
d) allowing said substrate to be converted by said biocatalyst thereby providing the (bio)chemical compound as the reaction product.

In preferred embodiments of the above-described aspects of the invention, the host cell is a bacterial cell, a plant cell, an insect cell, a mammalian cell, or another animal cell, such as birds and reptile cells.

In other preferred embodiments of the above-described aspects, the detector gene, i.e. gene to be activated or repressed by the interaction of said product with the regulatory gene, is a reporter gene, the expression of which causes the host cell to produce a detectable signal, such as a gene for a fluorogenic or chromogenic marker.

In yet another preferred embodiments of the above-described aspects, the detector gene is a selector gene, the expression of which permits host cells to grow under conditions where cells that do not express this same selector gene are not able to grow. A suitable example of such a gene is a gene for a resistance marker.

In still other preferred embodiments of the above-described aspects, the product-inducible expression of said detector gene is brought about by binding of said product to a regulatory protein and wherein said regulatory protein has the ability to bind to DNA and activate transcription from said at least one product-inducible expression system. Preferably, said regulatory element of said at least one product-inducible expression system is a binding site for said regulatory protein.

In still another preferred embodiment of the above-described aspects, the biocatalyst expression system is activated by an externally provided inducer.

In still a further preferred embodiment of the above-described aspects the at least one biocatalyst expression system comprises nucleic acid encoding at least two candidate biocatalysts each capable of catalyzing at least one reaction of a multi-step chemical conversion reaction. Preferably the product of the reaction catalyzed by one of said at least two candidate biocatalysts is the substrate for the reaction catalyzed by another of said at least two candidate biocatalysts and/or the nucleic acid encoding said at least two candidate biocatalysts is comprised on separate expression systems for the separate candidate biocatalysts.

### DESCRIPTION OF THE FIGURES

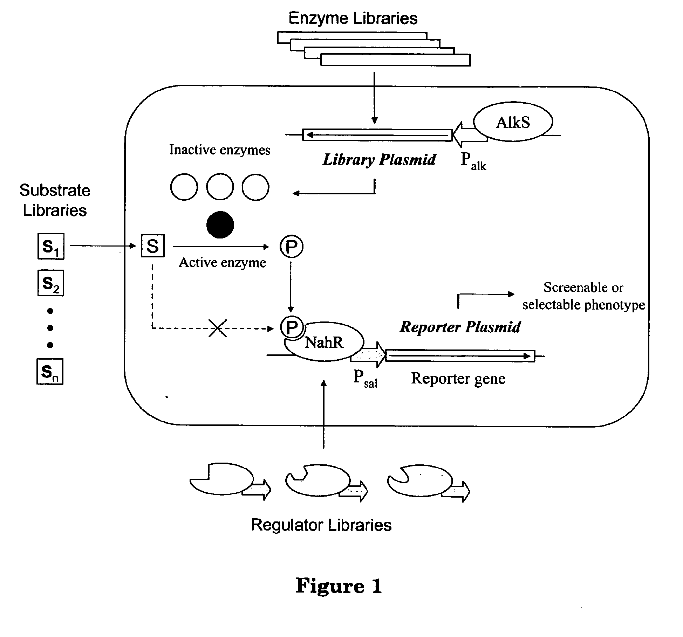

Figure 1 shows an illustrative example of the reporting and selection mechanisms used in aspects of the present invention in overview. In this example, bacterial cells express a library of potential enzymes (E₁, E₂, ...Eₙ), and substrate S is converted to desired product P in those cells that contain an enzyme capable of this conversion. A transcriptional regulatory protein that recognizes product P, but not the substrate S, then activates transcription of a detector gene. This allows identification of cells that contain active enzymes on the basis of a screenable or selectable cellular phenotype. The regulatory protein and the detector gene are herein encoded on a detector plasmid (herein also referred to as the detector expression systems).

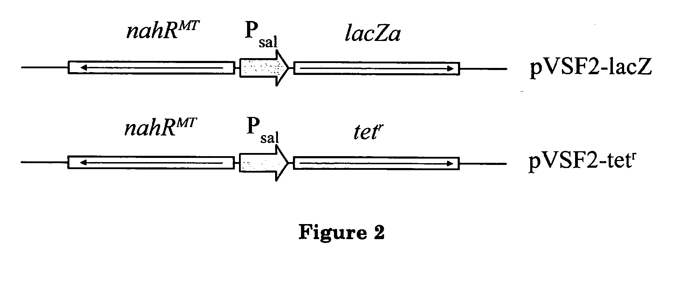

Figure 2 shows the reporter vector pVSF2-lacZ and selector vector pVSF2-tet^{r} that carry *lacZa* and *tetA* respectively, fused to the salicylate promoter, which are examples of a reporter gene expression system and a selector gene expression system respectively. The NahR mutant enables and regulates expression of the reporter and selector genes in the presence of product.

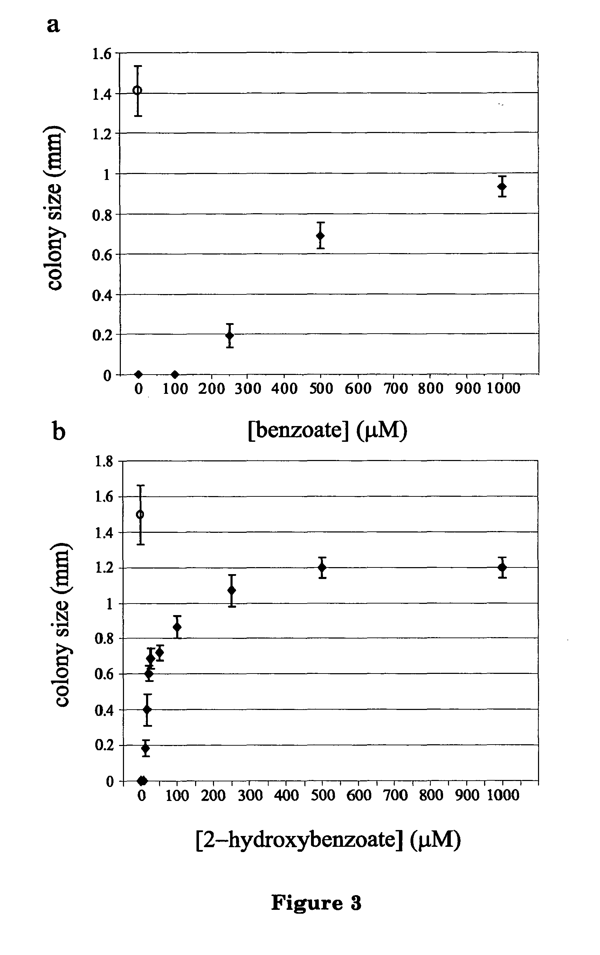

Figure 3 shows the effect of varying benzoate (a) and 2-hydroxybenzoate (b) concentrations on growth of DH10B-p VSF2-tet^{r}. Under non-selective conditions DH10B-pVSF2-tet^{r} is able to grow in the absence of an inducer (o), whereas under selective* conditions an inducer is required for growth (◆). Error bars indicate standard deviations. * note that the tetracycline concentrations for the benzoate and 2-hydroxybenzoate assays were 22.5 µg/ml and 25 µg/ml, respectively.

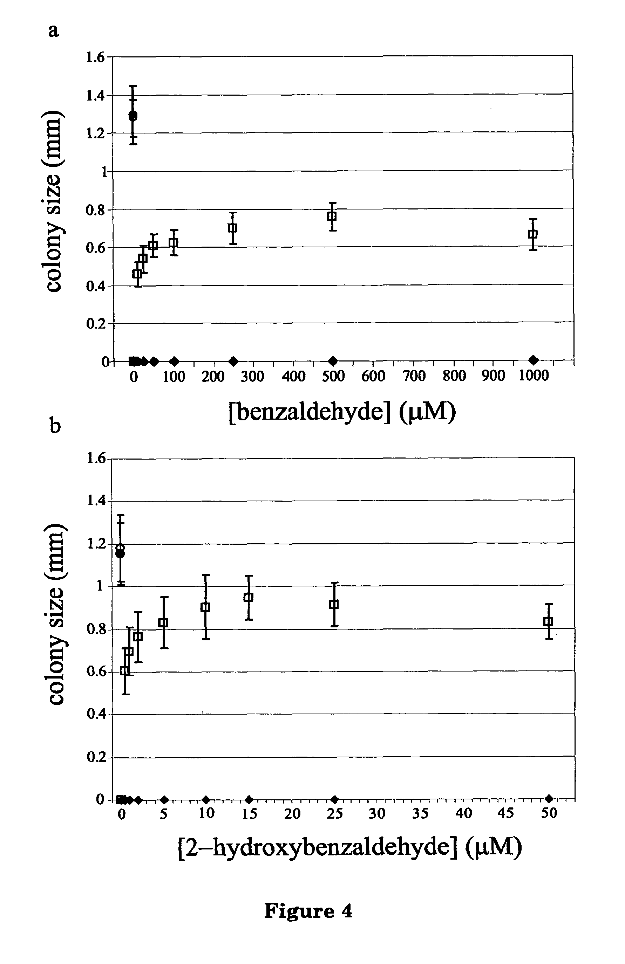

Figure 4 shows the effect of varying benzaldehyde (a) and 2-hydroxybenzaldehyde (b) concentrations on growth of cells containing an active enzyme. Under non-selective conditions cells with (o) and without (•) active enzyme are able to grow. Growth under selective* conditions requires an active enzyme and an aldehyde substrate. Cells containing an active enzyme are able to grow upon addition of benzaldehyde or 2-hydroxybenzaldehyde (□), whereas cells lacking an active enzyme fail to grow in the presence of benzaldehyde or 2-hydroxybenzaldehyde (◆). Error bars indicate standard deviations. * note that the tetracycline concentrations for the benzaldehyde and 2-hydroxybenzaldehyde assays were 22.5 µg/ml and 25 µg/ml, respectively.

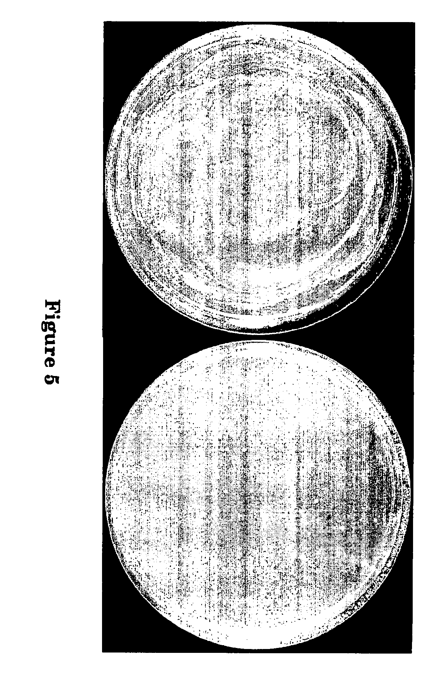

Figure 5 shows the selection of biocatalytically active cells from a background of inactive cells. Biocatalytically active cells that contain the active enzyme XylC were mixed with inactive cells in a ratio of 1:10⁵. About 10⁷ cells were plated and incubated on non-selective (left) and selective plates (right). Ten randomly chosen colonies from the selective plate were tested for XylC; all contained the plasmid.

### DETAILED DESCRIPTION OF THE INVENTION

### 1. Definitions

"Genetic engineering", "transformation" and "genetic modification" are all used herein as synonyms for the manipulation of DNA fragments by using techniques such as for instance restriction enzyme digestion, annealing and ligation and other techniques in order to produce new recombinant DNA fragments. The "recombinant" DNA may be ligated into an appropriate vector (e.g. plasmids, cosmids or viruses engineered for the purpose of accepting DNA insertions and replicating into a host cell) and the vector may be inserted into host cells which then reproduce the recombinant DNA fragments. The cloned material may be inserted into the chromosomal or genome DNA of a host cell or may reproduce extrachromosomally.

"Cloning" is referred to as the process of inserting DNA encoding a gene of interest into a vector, then establishing it as a stable part of a cell line.

The term "expression vector" as used herein refers to a relatively small DNA molecule that is used to introduce and express a specific gene into a target cell. Once the expression vector is inside the cell, the protein that is encoded by the gene is produced by the cellular transcription and translation machinery. Generally, these expression vectors include regulatory elements operably linked to the nucleic acid of the encoding specific gene (*i.e.* the nucleic acid for the candidate biocatalyst and/or the detector gene). "Transcription" refers to the synthesis of RNA on a DNA or RNA template. "Translation" refers to the synthesis of protein on the mRNA template. Nucleic acid is "operably linked" when it is placed into a functional relationship with another nucleic acid sequence. For example, a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. Enhancers do not have to be contiguous.

### 2. Basic system layout

The limited throughput possibilities of screening techniques can largely be avoided by using the herein described methods of detecting a biocatalyst. The working hypothesis that led to the present invention was based on the assumption that detection, based on selection, might be possible if host cell strains could be developed that use industrial products as essential regulators. This would permit the linkage of growth to the biocatalytic formation of product, thus signaling the presence of an intracellular biocatalyst capable of catalyzing a desired reaction. Such a linkage is created in the present invention by utilizing native or modified regulatory systems as sensing-transducing systems that detect the formation of products of interest, including chemicals that are or can be produced in industry. The general approach is to detect the presence of a cell-associated biocatalyst capable of catalyzing a desired reaction, by using a bacterial regulatory protein that recognizes the reaction product and relays this signal to one or more detector systems (based, for example, on a reporter gene expression system or on a selector gene expression system) (see Figure 1). With respect to the detection system, by coupling desired product detection to survival under selective conditions, the system links growth to intracellular formation of, preferably non-metabolized, biocatalysis products, enabling detection of a biocatalyst based on selective growth of biocatalytically active cells in a background of inactive cells. This basic concept was expanded to the present invention, which uses a host cell-associated and product-induced expression system, also termed the detector system herein, for indicating the presence of the sought-for biocatalyst.

The biocatalyst detection system as outlined herein may encompass a single host-cell system or it may encompass a system of multiple interacting cells. For instance, two cells may interact when they produce certain compounds wherein each cell carries out one step in a synthesis reaction. In such cases, the multi-step process of the present invention may be performed by two or more interacting cells wherein one utilizes the substrate as referred to herein and forms an intermediate compound which in turn functions as a substrate for another host cell that (also) comprises a biocatalyst for producing the required product. In this way intricate pathways for the formation of products may be formed.

Alternatively, said single steps in a synthesis reaction may be combined in single cells, resulting in newly developed biochemical pathways for the synthesis of desired end products.

### 3. The biocatalysis product

Methods of the prior art for detecting biocatalysts generally allow for the possibility of selecting cells that are capable of producing an essential nutrient, i.e. a medium component that, if omitted from a medium, prevents cell growth unless said cells have enzyme(s) to produce this component from other metabolites or other medium components. Such methods have been used for the identification of enzymes that produce an essential nutrient or growth medium component, such as prephenate (MacBeath *et al.,* 1998; Gamper *et al.,* 2000), pyruvate (Griffiths *et al.,* 2004) or ammonia (Robertson *et al.,* 2004). However, in those instances where a biocatalysis product is not an essential nutrient, or is not a metabolite, the methods of the prior art cannot be applied since cells that have the biocatalyst cannot not be selected over cells that do not. As a result, since many industrial chemical products are not essential growth medium components, biocatalysts suitable for the synthesis of said industrial chemical products cannot be selected on that basis.

By providing a product-inducible expression system comprising nucleic acid encoding at least one detector gene operably linked to a regulatory element, wherein the expression of said detector gene is directly or indirectly inducible by said product, the problem of the prior art methods may be overcome.

It is an advantage of the methods of the present invention that a biocatalyst for any biocatalytic chemical product may now be detected, selected and provided, as long as a chemosensor can be provided, i.e. in the form of a product-inducible expression system for a detector gene, that is capable if signalling the presence of the biocatalyst, via sensing of the product. The product-inducible expression system may sense a single product or a specific (group of) chemical(s).

Accordingly, a preferred embodiment of the present invention relates to products which are not essential growth medium components, a most preferred embodiment relates to products which are not natural metabolites of the host cell.

### 3.1. The detector system

The present invention uses a host cell-associated detector system to indicate the presence of the sought-for biocatalyst. The detector system may be based on any kind of biochemical cascade that indicates the occurrence of a cell-associated event in a single host cell, or even a series of host cells. Many such biochemical cascades are known in the art. The cell-associated event in the present case is the conversion of a substrate into a product by a biocatalyst and the resulting interaction of the product with an inducer of the detector system, the inducer for instance being a regulatory protein. Two exemplary detector systems that constitute preferred embodiments are described in more detail herein below: a) the reporter gene expression system and b) the selector gene expression system, which may be used individually or in combination in aspects of the present invention. Other detector systems may also be envisaged. The detector system may for instance also comprise a hybrid form of a reporter-selector system.

The product-inducible detector gene expression system may take any form possible. In principle, a large variety of systems is available to the person skilled in the art. The present invention was reduced to practice by providing a three-component signaling system comprising: 1) product; 2) product-inducible transcriptional regulator; and 3) transcription control element/promoter. As stated, other systems are within the scope of the present invention. A non-limiting list of alternative combinations for the three-component system is provided in Table 1 below.

### 3.2. Reporter detector system

The cell-associated detector system may in one preferred embodiment comprise a genetic expression system based on a reporter gene, in which case the detector system is based on the host-cell associated production of a detectable chemical substance, said substance generally being referred to as a marker. A preferred reporter expression system encompasses spontaneous or stimulated light emission, for instance through the expression of a gene for a fluorogenic or chromogenic marker. The purpose of the reporter gene is to allow detection, preferably visual detection, of cells that express the reporter gene either in colonies on a plate, or in other configurations of individual cells or microcolonies.

The reporter gene expression system may either be based on positive or negative signaling. For instance, in positive signaling, cells that contain the desired biocatalyst produce the detectable substance and are detected, and, *vice versa,* in negative signaling, cells that contain the desired biocatalyst do not produce the marker and may for instance be detected by elimination of marked cells. This principle will hold for many of the detector systems mentioned herein.

Many marker genes known in the art may be used as reporter genes in the context of the present invention, such as genes that encode enzymes which directly or indirectly contribute to chromogenic or fluorogenic characteristics. A preferred embodiment of a reporter gene is a gene for a fluorogenic or chromogenic marker and the reporter gene expression system is preferably based on positive signaling.

The reporter gene expression system used in aspects of the present invention suitably comprises a nucleic acid encoding at least one reporter gene. The system may comprise more than one such gene, but one reporter gene will generally suffice.

In general, other suitable methods for detection are based on a detectable change in the phenotype of the host cell which can include, for example, a change in enzyme activity, the onset of expression of soluble molecules or cell-surface molecules, cell cycle interference, modification of cell metabolism, etc.. The detector expression system may encompass induction, stimulation, acceleration, inhibition, blockage, or in general interference with for instance:
- cell mobility, such as chemotaxis;
- cell division, such as to block septation such that cells continue to divide without separating, thereby forming strand-like filaments or a system that blocks cell division to create maxi cells;
- cell metabolism, such as a system that accelerates metabolism by inducing a C-source utilization pathway that enables cells to outcompete other cells of the population;
- cell density, such as systems that produce intracellular low density biopolymers like medium-chain-lenth polyhydroxyalkanoates, which reduce the density of cells and make them float;
- cell surface molecules, such as a system expressing an antigen, antibody, binding protein, adhesin or other surface molecule causing cells to interact, coagulate, become attached to the growth vessel wall or a specific matrix material, sink to the bottom of the growth vessel or float to the top of the medium;
- product excretion, such as for instance selective excretion of biocatalyst, whereby the detector system couples a secretory leader sequence to the biocatalyst;
- pathogenicity, virulence

### 3.3. Selector detector system

The cell-associated detector system may in another preferred embodiment comprise a genetic expression system based on a selector gene, in which case the detector system is based on growth selection of the host-cell. The purpose of the selector gene is to allow the selection of one or more host cells that contain an active biocatalyst on the basis of the expression of said selector gene. This means that the host cell containing the biocatalyst may be selectively recovered by growth selection (i.e., by supporting the growth or preventing growth inhibition of cells that express the selector gene). Thus, the expression of a selector gene permits host cells to grow under conditions where cells that do not express this same selector gene are not able to grow. The skilled person will understand that the selector gene-based detector system may sometimes be preferred over the reporter gene-based detector system because of its relative ease with which cells that contain the desired biocatalyst can be enriched and purified.

The selector gene-based detector system used in aspects of the present invention suitably comprises a nucleic acid encoding at least one selector gene. The system may comprise more than one such gene, but one selector gene will generally suffice. A wide variety of selector genes known in the art are suitable for use in the present invention. Such genes include, but are not limited to, genes for antibiotic resistance; genes that complement an auxotrophy in the host cell, or any other conditionally lethal or growth-inhibiting mutation; and other genes that allow growth under specific conditions that do not permit growth of host cells that do not contain or express these same genes. Such conditions include, but are not limited to, the inability of host cells to utilize various possible C-sources, the presence in the growth medium of toxic chemicals, protein toxins, toxic metals, or growth conditions which include extremes of temperature, pH, ionic strength, that is, conditions outside of the normal ranges for each of these characteristics for the said host cells.

It will be understood that the product-inducible expression of the detector gene may be brought about by positive control (e.g. binding of the desired product - which acts as an inducer - to an activator protein) or by negative control (e.g., binding of the product - which in this example acts as a repressor - to a repressor protein), in which case, for instance, a property that prevents cell growth is removed, allowing growth of those cells who are able to produce a compound that causes repression.. Thus, there may be activation of a system needed for growth, or repression of a system that blocks growth, both of which result in cell growth under conditions that prevent growth of cells not producing the relevant desired product.

Suitable selection detector systems broadly include genes that allow discrimination between growth of host cells under specific conditions and a lack of growth of host cells in the absence of these specific conditions. For induction based regulatory systems, induction is coupled to growth by introducing conditions that enable growth. For repression based regulatory systems, repression is coupled to growth by removing specific conditions that block growth. Said specific conditions include the inability of host cells to utilize various possible C-sources, the presence in the growth medium of toxic chemicals, protein toxins, toxic metals, or physical growth conditions of temperature, pH, ionic strength, radiation, pressure or other conditions, outside of the normal ranges for each of these characteristics for the said host cells.

Preferred selection detector systems include:
- genes for antibiotic resistance;
- genes that complement an auxotrophy in the host cell, or any other conditionally lethal or growth-inhibiting mutation;

Most preferably, the host cell population from which a desired host cell or cells are to be selected are grown in the presence of a suitable antibiotic, and the selector gene is a gene for resistance to said antibiotic, since this will result in the selective outgrowth and thus in the selective enrichment of the host cell or cells of interest.

### 3.4. Regulatory elements and the expression vector

The detector genes comprised in the detector expression systems are preferably operably linked to one or more regulatory elements. For instance, the detector gene may be operably linked to an inducible promoter for initiation of its transcription. Thus, the detector systems may comprise the nucleic acid encoding the detector gene as well as optional regulatory elements in the form of expression systems, which expression systems may in turn be in the form of an expression cassette, which comprises detector gene and regulatory elements in the form of a nucleic acid construct.

The term "regulatory element" as used herein is synonymous with such terms as "regulatory sequence", "control sequence", "transcription-control element", "transcription-control region" "transcription-control sequence" and "transcriptional and translational regulatory nucleic acid". The term "regulatory element" refers to one or more DNA sequences necessary for the expression of an operably linked coding (gene) sequence in a particular host cell. The control sequences that are suitable for prokaryotes, for example, include a promoter, optionally an operator sequence, and a ribosome binding site. Eukaryotic cells are known to utilize promoters, polyadenylation signals, promoter-proximal elements, upstream activating sequences (UASs), and enhancers. In general, the regulatory elements may include, but are not limited to, promoter sequences, ribosomal binding sites, transcriptional start and stop sequences, translational start and stop sequences, and enhancer or activator sequences. A "promoter" is a region of DNA involved in binding of RNA polymerase in order to initiate transcription. "Enhancers" can stimulate transcription from a promoter tens of thousands of base pairs away. "Promoter-proximal elements", generally lose their influence when moved further from the promoter. The regulatory elements will generally be appropriate to the host cell used to express the genes of interest; for example, transcriptional and translational regulatory nucleic acid sequences from *Bacillus* are preferably used to express genes in *Bacillus.*

The expression cassettes may be transferred to an appropriate host cell by using an expression vector. Numerous types of appropriate expression vectors, and suitable regulatory sequences are known in the art for a variety of host cells. In a preferred embodiment, the regulatory elements include a promoter and transcriptional start and stop sequences. Promoter sequences as used herein may encode either constitutive or inducible promoters unless expressly stated otherwise. The promoters may be either naturally occurring promoters or hybrid promoters. Hybrid promoters, which combine elements of more than one promoter, are also known in the art, and are useful in the present invention. The salicylate promoter as used for inducing transcription of the selector gene expression system in the Examples may for instance be used. The skilled person is capable of identifying other promoters suitable for obtaining adequate expression of the reporter or selector gene. Adequate expression may for instance be obtained by controlling the copy number of the vector on which the promoter and the selector gene are placed

In choosing an appropriate promoter for activation of transcription, the skilled person is aware that adequate expression can usually be obtained with promoters whose expression levels are controlled by regulatory proteins. Such regulatory proteins may serve as "chemosensors" in relaying the presence of the product to the detector system as will now be described.

According to the present invention, the expression of the genes in the detector system is inducible by the product. This may in principle be realized by any method available to the skilled person. One way of achieving this is described in detail below. This exemplary procedure constitutes a mere preferred embodiment of the invention. In this preferred embodiment, the product-inducible expression of the detector gene is brought about by binding of the product to a regulatory protein that has the ability to bind to DNA and activate transcription of the detector gene in the corresponding expression system.

In general, "regulatory proteins" bind to "regulatory elements". More precisely, "regulatory elements" are binding sites for "regulatory proteins". The term "regulatory protein" as used herein is synonymous with the terms "transcription factor", "transcriptional regulator" or "transcriptional activator protein" which are proteins capable of binding to a regulatory element and as a result thereof either stimulate or repress transcription. Both stimulatory and repressive forms of regulatory proteins may be used in aspects of the present invention. Thus, in a preferred embodiment, the regulatory element of the reporter or selector expression system is a binding site for a regulatory protein.

The regulatory protein is the preferred "chemosensor" for sensing the presence of the product. The specific physical interaction between product and regulatory protein provides a very suitably mechanism for product-inducible activation of transcription of the detector expression system. Very suitable regulatory proteins are transcriptional regulators, such as the transcriptional activator protein NahR from *P. putida,* a LysR-type transcriptional regulator. Even more suitable are mutants of such transcriptional regulator proteins with a broader "inducer range", i.e. which recognize more products as possible inducers for transcription activation.

Although inducible expression of the gene for the regulatory protein is equally suitable, the skilled person will appreciate that the regulatory protein is preferably encoded by a constitutively expressed gene, as it is convenient that the components of the product-inducible detector expression system are available at sufficient levels inside the host cell, so that the expression of the detector gene will start immediately upon first appearance of product. Nonetheless, the regulatory protein may be encoded by a an inducible gene, i.e. a gene which is under control of a chemical inducer or one or more of a set of environmental inducing conditions, such as temperature, pressure, ionic strength or cell crowding.

Preferably, the amount of regulatory protein is adequate for ensuring the regulatory function, i.e. for ensuring that the system responds to a suitable range of product concentrations. The expression of the regulatory protein may be brought about by providing the host cell with a suitable expression system. If needed, a system for the expression of the regulatory protein is preferably provided for on a vector already used for introducing an expression system in the host cell, although a separate vector may also be used for introducing the system for the expression of the regulatory protein in the host cell. To provide the regulatory protein its encoding gene may be placed on i) the vector containing the detector gene(s) ii) the vector containing the biocatalyst library iii) a separate vector with solely this purpose, iv) a separate vector with other functions or purposes or, v) the chromosome of the host cell.

Thus, the regulatory protein used for sensing/transducing the presence of the product and its cognate promoter are most suitably used in as a pair, in which case the choice of the promoter may depend on the choice of the regulatory protein that recognizes the product (see also Table 1).

**Table 1. Non-extensive list of suitable combinations of product - transcriptional regulator - promoter combinations.**

| **Typical Products that can be detected** | **Transcriptional Regulator (wildtype or characterized mutant)** | **Promoter** | **Reference** |
|---|---|---|---|
| • unsubstituted and substituted benzoic acids | NahR | Salicylate promoter (Pₛₐₗ) | {Cebolla, 1997 #25;Park, 2005 #143} |
| • salicylamide | | | |
| • unsubstituted and substituted benzoic acids | XylS | *meta* pathway operon promoter (Pₘ) | {Ramos, 1986 #157}{Ramos, 1990 #42} |
| • unsubstituted and substituted toluenes, benzyl alcohols and benzaldehydes | XylR, | Upper pathway operom promoter (Pᵤ) | {Ramos, 1990 #42}{Skarfstad, 2000 #141}{Garmend ia,2001 #41}{Abril, 1989 #181} |
| • phenol | | | |
| • benzene | | | |
| • biphenyl | | | |
| • unsubstituted and substituted phenols | DmpR | Phenol catabolic operon promoter (Pₒ) | {Skarfstad, 2000 #141}{Wise, 2000 #54} |
| • tetracycline | TetR | *tetO* promoter | {Scholz, 2003 #148}{Henssler, 2004 #147}{Bertram, 2005 #146} |
| • tetracycline analogs | | | |
| • tryptophan, 5-methyltryptophan (co-repressors) | TrpR | Tryptophan promoter (Pₜᵣₚ) | {Arvidson, 1991 #182}{Chevalet, 2000 #183} |
| • 3-β-indoleacrylic acid (inducer) | | | |
| • linear alkanes | AlkS | *alkB* promoter (P_{alkB}) | {Smits, 2001 #26} |
| • dicyclopropylketone | | | |
| • toluene | TodST (two-component regulatory system consisting of a sensory histidine kinase and response regulator) | *todX* promoter | {Tropel, 2004 #117} |
| • styrene | StySR (two-component regulatory system consisting of a sensory histidine kinase and response regulator) | *styA* promoter | {Tropel, 2004 #117} |
| • several cationic lipophilic compounds such as tetraphenylphosphonium, crystal violet, rhodamine 6G | BmrR | *bmr* promoter | {Schumacher Maria, 2002 #134} |
| • several quaternary ammonium compounds such as benzalkonium, methyl green, ethidium, proflavine, propamidine palmatine, avicin, palmatine, chlorhexidine | QacR | *qacA* promoter | {Schumacher Maria, 2002 #134}{Grkovic, 2003 #149} |
| • L-arabinose | AraC | Arabinose operon promoter (P_{BAD}) | {Daunert, 2000 #52} |
| • L-mannose | | | |
| • arsenite, arsenate, antimonite | ArsR | *ars* promoter | {Daunert, 2000 #52} |

Promoters may initiate the transcription of any reporter or selector gene placed downstream from it. A non-limiting list of suitable detector genes is provided in Table 2.

**Table 2. Examples of detector genes suitable for the construction of detector systems in the present invention.**

| **Detector gene** | **Phenotype/Indication** | **Required conditions** | **Type of marker** | **Reference** |
|---|---|---|---|---|
| *tetA* | tetracycline resistance | growth medium containing tetracycline | resistance | {Schnapping er, 1996 #186} |
| *kan* | kanamycin resistance | growth medium containing kanamycin | resistance | {Wright, 2005 #187} |
| *cat* | chloramphenicol resistance | growth medium containing chloramphenicol | resistance | {Wright, 2005 #187} |
| *LacZa* | β-galactosidase | 5-bromo-4-chloro-3-indolyl-beta-D-galactopyranoside (β-galactosidase substrate) | chromogenic | {Daunert, 2000 #52} |
| *luxCDABE* | bacterialluciferase | - | bioluminescent | {Hakkila, 2002 #185} |
| *lucFF* | firefly luciferase | luciferin (firefly luciferase substrate) | bioluminescent | {Hakkila, 2002 #185} |
| *gfp* | green fluorescent protein | - | fluorophoric | {Hakkila, 2002 #185} |
| *dsred* | red fluorescent protein | - | fluorophoric | {Hakkila, 2002 #185} |
| genes complementing an auxotrophy in the host (e.g. chorismate mutase) | complementation of an auxotrophy in the host (e.g. restoring phenylalanine and tyrosine synthesis pathway) | auxotrophic host (e.g. chorismate knock-out strain) | protein essential for survival and growth of host cell | {Gamper, 2000 #13}{Griffith s, 2004 #23} |
| genes allowing the host to grow on particular carbon or nitrogen source (e.g. amidase) | growth on minimal media (e.g. amidase) | minimal media containing carbon or nitrogen source only degradable by marker protein (e.g. degradable amide) | protein essential for survival and growth of host cell | {Gabor, 2004 #108} |

Either of the expression systems mentioned herein (the detector expression system and the biocatalyst expression system) may suitably be provided in the form of a nucleic acid construct or expression cassette, which in turn may be comprised in a suitable vector for transfer to the host cell. Although it may be most convenient from the viewpoint of a developer to work with separate vectors for each system (for instance, wherein the expression systems are each provided in the form of separate plasmids), it is also possible to provide both expression systems on a single vector. The vectors are hereinafter referred to in singular form (a/the vector), although it is intended that a plural form (the vectors) is also included by that term. The expression vector may be either a self-replicating extrachromosomal vector or a vector which integrates into a host genome. The expression vector may comprise additional elements. For example, the expression vector may have two replication systems, thus allowing it to be maintained in two organisms, for example in mammalian or insect cells for expression and in a prokaryotic host for cloning and amplification. Furthermore, for integrating the expression vector, the expression vector may contain at least one sequence homologous to the host cell genome, and preferably two homologous sequences which flank the expression constructs. The integrating vector may be directed to a specific locus in the host cell by selecting the appropriate homologous sequences for inclusion in the vector. Constructs for integrating vectors are well known in the art. Either of the expression systems may include a marker gene (an additional marker gene in the case of the selector gene expression system, which system already comprises one selectable marker gene), operably linked to a regulatory element (such as a promoter), for the purpose of allowing the assessment of successful host cell transformation. Suitable marker genes for cell transformation purpose are non limiting and well known in the art. A proper reference handbook for molecular cloning techniques and suitable expression vectors is Sambrook and Russell (2001). Minimum requirements are an origin of replication, a regulatable promoter and preferably multiple cloning sites. With PCR and DNA synthesis all desired elements can be introduced, combined, and arranged at will for optimal functionality of the vector.

### 4. The biocatalyst

The term "biocatalyst" is used herein to refer to a biochemical catalyst that activates or accelerates a chemical (conversion) reaction. In many instances, the biocatalyst will be an enzyme, however, it may also be a complex of enzymes, a combination of an enzyme and a co-factor, etc. The term is intended to cover all forms of the compound(s), both in the form of a protein, possibly modified by covalent or non-covalent attachment of sugars, oligosaccharides, polysaccharides or fatty acids, lipids, steroids and other chemical substituents and compounds, in the form of a nucleic acid or in the form of a combination of several such compounds. Generally, the true nature of the biocatalyst may first be established or assessed after its selection in a method of the present invention.

The population of candidate biocatalysts from which a biocatalyst is to be selected in a method of the present invention is very suitably provided in the form of a library of candidate nucleic acids. Such nucleic acids may comprise much more information than that encoding the biocatalyst protein itself. However, this is of no consequence to its possible selection. A "library" can be produced by digesting all DNA from a suitable source organism with a restriction enzyme and cloning the fragments into a vector, which will result in the production of many different recombinant vectors, each with a different fragment of DNA cloned into it. The collection of many different recombinant vectors together forms the "library". The library may be in the form wherein it is comprised in host cells or viruses, which facilitates its propagation or maintenance. The library may be produced by any method available to the skilled person, for instance by shotgun cloning. "Shotgun cloning" is referred to as using the whole genome of an organism as the starting point for cloning. Alternative, DNA/RNA can be isolated from the environment, resulting in thousands of different genes which may have derived from many different organisms; such a collection of nucleic acid material is referred to as a metagenome. At the other end of the spectrum, specific libraries may be constructed that contain only genes expected to encode the desired biocatalysts, based for instance on DNA homologies, previous screening experiments, or commercially available libraries. A library constructed by shotgun cloning techniques may contain from a few thousand (microorganisms) to hundreds of thousands (higher eukaryotes or microbial metagenomes) different recombinant plasmids while only one or a few harbor a sequence of interest. In order to reduce the size of libraries derived from eukaryotic organisms, it is sometimes advantageous to produce cDNA libraries. Since protein coding regions generally account for only a small percentage of the total genome size of eukaryotic organisms and since eukaryotic cells generally express only a subset of their genes, the production of cDNA copies of the cellular mRNA using reverse transcriptase and the cloning thereof into a cDNA library results in a library of reduced size comprising only the part of the organism's genome that is of most interest (the protein coding sequences that are expressed). cDNA library construction is well known to the skilled person. Other library types include a library obtained by i) mutation (directed or random) of a single parent gene ii) recombination of several parent genes (such as libraries produced by gene shuffling) iii) a combination thereof.

The "library" may be a library of genes or a library of organisms.

In the case the library is a library of organisms, the host may already have the desired biocatalytic activity, and the aim of the invention is to develop an improved biocatalyst. The population of candidate biocatalysts may for instance be provided in the form of a mixed population of potentially biocatalytically active bacterial cells, i.e. cells of different strains, for instance isolated from a soil or water sample, to which cells the reporter or selector systems are added, in order to select the best strain.

In the case the library is a library of genes, these genes may be cloned from a source organism. Alternatively, the library of genes may be generated through mutation of a single parent gene or by cutting genome(s) into fragments and inserting this into an expression vector.

The individual genes that together form the library or population of candidate biocatalysts may be encoded on chromosomal or episomal DNA (i.e. on a plasmid or other vector)

They may be expressed constitutively; or they may be on an inducible expression vector, in which case they are expressed as a result of activation through one of various possible operably linked regulatory elements. In fact it is not limiting how and if the biocatalyst expression is induced. Expression may be constitutive, it might be under control of a native regulator, either known or not yet known, or it might be part of a library under control of a specifically designed induction system. Regardless of the way in which the biocatalyst gene(s) is/are expressed, it is preferred that expression is sufficient to result in detectable (reporter or selector based) biocatalyst activity. The sufficiency of the biocatalyst activity is ultimately determined by the sufficiency of product formation. The activity of the candidate biocatalysts may range from non-detectable to detectable.

The biocatalyst expression system may in general have the same constitution as the detector expression system described in more detail above. For expression, the biocatalyst gene will normally be operably linked to a regulatory element. In analogy to the detector expression system, the expression of biocatalyst in the biocatalyst expression system may be constitutive or inducible by a suitable inducer. Either option may readily be realized by the skilled person through selection of a suitable promoter. In a preferred embodiment of the aspects of the invention, the biocatalyst expression system is activated by an externally provided inducer. For instance, the expression of the biocatalyst nucleic acid may be under the control of the AlkS protein, a transcriptional regulator which, in the presence of alkanes, activates the expression of the Palk promoter. In such instances, the biocatalyst expression system should also comprise the *AlkS* gene (including its own promoter). There are many more expression systems available to the skilled person. An exemplary list is provided in Table 3 below. Inducers for the various inducible expression systems used in aspects of the present invention may, but need not necessarily differ from one another.

**Table 3. Examples of suitable biocatalyst or regulator protein expression systems.**

| **Promoter** | **Transcriptional regulator** | **Inducible/ Constitutive** | **Reference** |
|---|---|---|---|
| *alkB* promoter (P_{alkB}) | AlkS | inducible | {Smits, 2001 #26} |
| *lac* promoter (P_{lac}) | LacI | inducible | {Hu, 1987 #192} |
| tryptophan promoter (Pₜᵣₚ) | TrpR | inducible | {Chevalet, 2000 #183} |
| trc promoter (P_{trc}) | LacI | inducible | {Amann, 1983 #189} |
| salicyalte promoter | NahR | inducible | {Yen, 1991 #188}{Gamper, 2000 #13} |
| T7 promoter | LacI | inducible | {Studier, 1990 #190} |

It will be understood that the expression of the biocatalyst must be such that the level of production of the product will not result in the premature death of the host cell. To circumvent this problem, the expression of the biocatalyst may for instance be controllable, for which reason the biocatalyst expression is preferably inducible, or the host cell may be capable of excreting the product, or the product may be converted to a less harmful compound, or it may be degraded. Alternatively, by lowering the concentration of the substrate in the medium, the amount of product that can be formed is also lowered, and conditions are then sought that limit the amount of product made to a level that permits detection or selection of cells that contain the desired biocatalyst, but do not result in cell death. The net effect of any of the foregoing or similar measures should be that a biocatalyst can be identified that does not result in damage to the cell that contains the biocatalyst.

In a further preferred embodiment of the above-described aspects at least one biocatalyst expression system may comprise the coding nucleic acid for at least two candidate biocatalysts, each capable of catalyzing at least one reaction of a multi-step chemical conversion reaction. Preferably, the product of the reaction catalyzed by one candidate biocatalyst is the substrate for the reaction catalyzed by another candidate biocatalyst. In this way complete biochemical pathways may be uncovered and specific biocatalysts selected for each reaction step. In another embodiment, nucleic acid encoding the various candidate biocatalysts may be provided in separate expression systems, each expression system facilitating the expression of a separate candidate biocatalysts. Also in this manner, multi-step chemical conversion reactions may be provided for.

It will be understood that the host organism may or may not itself harbour enzymes and optional co-factors for catalyzing an intermediate reaction from a multi-step reaction in which the candidate biocatalyst catalyzes the last step of converting the final intermediate compound into the desired product. The intermediate compound may be a natural intermediate in the host cell, for the production of which the host cell likely has available an enzyme or a set of enzymes. Alternatively, the candidate biocatalyst may produce an intermediate compound, which can be converted into a product by an enzyme naturally encoded for and expressed (i.e. available) in the host organism under the conditions provided. Thus, the candidate biocatalyst need not necessarily catalyze the final reaction in a multi-step reaction process. It should be noted that in such instances, the enzyme system present in the host cell that contributes towards forming the inducer for activating transcription of the detector gene in such a multi-step system, may be encoded on host cell chromosomal or episomal DNA, including possibly plasmid DNA that also encodes the product-inducible expression system itself. Thus, apart from potentially catalysing the final reaction towards formation of the product, the candidate biocatalyst as defined herein may also catalyze the critical reaction in a possible chain of reactions of a multi-step reaction process, which critical reaction is the sought after reaction that ultimately leads (possibly via additional reaction steps) to the production of the sought after product.

The chemical conversion reaction may be any chemical reaction which benefits from activation or acceleration by a biocatalyst. Reaction types that can be catalyzed by the candidate biocatalyst include but are not limited to enzymes of each of the major enzyme classes and subclasses:

### 1. Oxidoreductases.

1. 1. Acting on the CH-OH group of donors.
1. 2. Acting on the aldehyde or oxo group of donors.
1. 3. Acting on the CH-CH group of donors.
1. 4. Acting on the CH-NH(2) group of donors.
1. 5. Acting on the CH-NH group of donors.
1. 6. Acting on NADH or NADPH.
1. 7. Acting on other nitrogenous compounds as donors.
1. 8. Acting on a sulfur group of donors.
1. 9. Acting on a heme group of donors.
1. 10. Acting on diphenols and related substances as donors.
1. 11. Acting on a peroxide as acceptor (peroxidases).
1. 12. Acting on hydrogen as donor.
1. 13. Acting on single donors with incorporation of molecular oxygen.
1. 14. Acting on paired donors, with incorporation or reduction of
1. 15. Acting on superoxide as acceptor.
1. 16. Oxidizing metal ions.
1. 17. Acting on CH or CH(2) groups.
1. 18. Acting on iron-sulfur proteins as donors.
1. 19. Acting on reduced flavodoxin as donor.
1. 20. Acting on phosphorus or arsenic in donors.
1. 21. Acting on x-H and y-H to form an x-y bond.
1. 22. Other oxidoreductases.

### 2. Transferases.

2.1. Transferring one-carbon groups.
2.2. Transferring aldehyde or ketone residues.
2.3. Acyltransferases.
2.4. Glycosyltransferases.
2.5. Transferring alkyl or aryl groups, other than methyl groups.
2.6. Transferring nitrogenous groups.
2.7. Transferring phosphorous-containing groups.
2.8. Transferring sulfur-containing groups.
2.9. Transferring selenium-containing groups.

### 3. Hydrolases.

3.1. Acting on ester bonds.
3.2. Glycosylases.
3.3. Acting on ether bonds.
3.4. Acting on peptide bonds (peptide hydrolases).
3.5. Acting on carbon-nitrogen bonds, other than peptide bonds.
3.6. Acting on acid anhydrides.
3.7. Acting on carbon-carbon bonds.
3.8. Acting on halide bonds.
3.9. Acting on phosphorus-nitrogen bonds.
3.10. Acting on sulfur-nitrogen bonds.
3.11. Acting on carbon-phosphorus bonds.
3.12. Acting on sulfur-sulfur bonds.
3.13. Acting on carbon-sulfur bonds.

### 4. Lyases.

4.1. Carbon-carbon lyases.
4.2. Carbon-oxygen lyases.
4.3. Carbon-nitrogen lyases.
4.4. Carbon-sulfur lyases.
4.5. Carbon-halide lyases.
4.6. Phosphorus-oxygen lyases.
4.7. Other lyases.

### 5. Isomerases.

5.1. Racemases and epimerases.
5.2. Cis-trans-isomerases.
5.3. Intramolecular oxidoreductases.
5.4. Intramolecular transferases (mutases).
5.5. Intramolecular lyases.
5.6. Other isomerases.

### 6. Ligases.

6.1. Forming carbon-oxygen bonds.
6.2. Forming carbon-sulfur bonds.
6.3. Forming carbon-nitrogen bonds.
6.4. Forming carbon-carbon bonds.
6.5. Forming phosphoric ester bonds.
6.6. Forming nitrogen-metal bonds.

It is an aim of the present invention to provide for biocatalysts capable of producing a product that represents the desired isomer or has the desired chirality. Isomerism is the property of compounds with identical molecular formulae but differing in the nature or the sequence of bonding of their atoms or in the arrangement of their atoms in space. Chirality is the property of a compound being non-superimposable on its mirror image due to the presence of a carbon atom bonded to four nonidentical substituents (a chiral center). With respect to chirality and stereoisomers that are non-superimposable mirror images, the product may be R or S or a racemic mixture of enantiomers. With respect to stereoisomers that are not mirror images of one another, the product may be in the form of individual diastereomers or as a diastereomeric mixture, wherein the compound(s) may have *cis, trans,* Z or E configuration when a rotation hindering double bond is present. For more information on production of isomers, see reference handbooks on the subject (*e.g.* Williams, 1999; Noyori, 1994).

It will be understood that once a biocatalyst is detected via detection of the host cell in which it is produced, it may be selected,and ultimately isolated from the host organism by methods known in the art *per se.* Alternatively, as stated, the detector expression system may be in the form of a biocatalyst secretion system, wherein the nucleic acid that encodes the candidate biocatalyst is fused to a secretory leader sequence. Upon its secretion, the biocatalyst may then be isolated directly from the test medium. In all cases the biocatalysts may be isolated and purified if desired using conventional techniques, including but not limited to filtration, precipitation, crystallization, chromatography, and the like. The biocatalysts may be characterized using conventional means, including methods to determine biocatalytic properties (kinetic data, substrate specificity and substrate range) and physicochemical properties such as solubility and stability data and spectral data.

### 5.1. The host cell

The term "host cell" as used herein refers to the cell with which the detector system for indicating the presence of the sought-for biocatalyst is associated. The host cell of the present invention may be any host cell capable of providing the cellular transcription and translation machinery required for expression of the population of candidate biocatalysts and of the necessary components of the detector systems. One very advantageous host cell for this purpose is the host cell in which the vectors comprising the library of biocatalyst genes is maintained. However, in an alternative, host cells that are better equipped for the expression of the phenotype brought about by the detector gene may also be used. For instance if a suitable host cell is available that comprises a suitable product-inducible expression system for a detector gene, it may be easiest to introduce into said host cell the biocatalyst expression system. It is also foreseeable that both detector expression systems and the biocatalyst expression system are introduced into a host cell separately. All these embodiments are envisioned in aspects of the invention. The choice for the host cell may suitably be based on the compatibility of expression vectors with the host and the availability of suitable regulatory elements. Most important however, is the availability in a host cell line or compatibility with a host cell line of a product-inducible gene expression system wherein the product of interest activates or represses transcription of the detector gene.

The host cell may be a micro-organism, such as a prokaryotic micro-organism or a eukaryotic micro-organism, a plant cell, or an animal cell line. Suitable prokaryotes include both archaea and bacteria. Suitable eukaryotic micro-organisms include yeast cells, fungi or protist cells. Suitable animal cell lines are for instance insect cell lines or cells of birds, reptiles, and fish, but also mammalian (including human) cell lines may be used. Preferred host cells are bacterial host cell lines because the bacterial metabolism provides for the simplest metabolic environment wherein the inducer function of the product is least likely to be disturbed.

The term "capable of facilitating or permitting access of said substrate to said biocatalyst" means that the host cell must be capable of enabling the substrate to access the sought for biocatalyst such that contact between substrate and biocatalyst can be made. This generally implies that the substrate must pass through the cell envelope or membrane system (a process generally referred to as "substrate uptake") to reach a biocatalyst that resides within the cytoplasm or other cell compartments. (Bacterial) cells can be modified to improve uptake of larger molecules. However, biocatalysts may also be located in plasma or cytoplasmic cell membranes or even outside these membranes in periplasmic or other extracytoplasmic spaces. In those cases substrates may have access to biocatalysts without actually passing through cell membranes. An exemplary category of detector proteins in the periplasmic space are the chemotactic receptors. These recognize a wide range of compounds, may be modified to recognize new compounds, and are known to transfer signals across the cytoplasmic membrane, switching on motility genes (or other selectable genes). Chemotaxis should be understood as being a possible detection method as referred to herein, as properly modified cells may "swim" towards the substrate in case they are capable of converting it to a product recognized by the chemotactic receptor.

### 5.2. The host-cell's test or growth environment

The choice for a particular detector gene will require the provision of a corresponding test environment or selective growth environment. For instance in the case of an antibiotic resistance gene, such as for instance the tetracycline resistance gene *tetA*, the provision in the growth medium of the antibiotic tetracycline is essential; whereas in the case of a chromogenic gene, such as for instance the *lacZ* gene, the provision in the growth medium of X-Gal is essential, in order to permit detection of the desired phenotype.

Test media for assaying the various detector gene expression systems are well known in the art and generally include pH buffers and a suitable aqueous saline base.

### 6. The substrate

The term "substrate" as used herein refers to the reactant in the biocatalyst-catalyzed reaction, i.e. the chemical compound that interacts with the active site of the biocatalyst and is converted to a product, preferably (directly into) the desired product.

As noted above, in order for a substrate added to the cell's external environment to come into contact with the candidate biocatalyst, the host cell may comprise some sort of specialized or general uptake system for transporting the substrate across the cell membrane in order to take up the substrate. However, as stated this is not essential as simple diffusion of the substrate across the cell membrane into the intracellular environment is also envisioned, as is conversion in extracytoplasmic spaces or even in membranes as described above. In such cases vectorial transport systems may for instance be present, where a single membrane located protein system carries out transmembrane transport while converting the substrate to a product. As a result, the substrate emerges in the cytoplasm in the form of product. How a cell will take up a given substrate depends on the availability or absence of specific or general uptake systems encoded by genes located on cellular chromosomes or on extrachromosomal DNA. Furthermore, substrate transport through cellular membranes also depends on the chemical properties of the substrate itself (*e.g.* hydrophobicity, ionic character) and the permeability of the host cell membrane. It should be understood that if the biocatalyst is produced in a certain cell compartment, it is essential that the substrate can be in direct physical contact with the biocatalyst so that conversion into the product can take place. In analogy, the product should be capable of inducing the activation of detector gene transcription, so that direct physical contact between the various essential compounds must be possible. The lack of or limited compartmentation of the prokaryote cell, also therefore makes such a cell very suitable as a host cell in aspects of the inventions.

The substrate in aspects of the invention is chosen such that it is the most likely candidate for being converted into the desired product. In the examples provided herein benzaldehyde was chosen as a substrate for the production of benzoate. The skilled person is capable of selecting a substrate such that it can be taken up by the cell and provides for a suitable candidate substrate for the sought after conversion reaction. Of aid in selecting suitable substrates may be information relating to metabolic pathways and to information on the biosynthesis of compounds found in various biological organisms, such as microorganisms, animals and plants (see for instance the KEGG databases, such as KEGG Pathway, KEGG genes and KEGG Ligand at http://www.genome.ad.jp/kegg/kegg2.html).

Also of aid in selecting suitable substrates may be schemes for preparation of (organic) chemical compounds which are used in methods known to those skilled in the art following procedures set forth in handbook references such as Fieser and Fieser, 1991 or the series Organic Reactions (John Wiley & Sons, New York, (1942-), Volumes 1-40). Modifications to the conversion reaction may be made and will be suggested to one skilled in the art having referred to the disclosure contained in this application.

The substrates used in the sought after biocatalyst conversion reaction for preparing the product compound generally are either available from commercial suppliers, such as Aldrich Chemical Co., or may be prepared by methods known to the skilled person, including the procedures described herein.

The test substrate may suitably be provided in the form of a pool of candidate substrates for one or more possible given reactions, in order to assess the presence of a suitable substrate for the reaction, which substrate can be converted into one or more products detected by the sensing component of the product-inducible detector system. In fact, a complete library of potential substrate compounds may be added to the test or growth medium, preferably in the form of defined groups, so that one or more suitable substrates can be detected and/or selected.

It will be appreciated that this method for finding a suitable substrate, may optionally be performed simultaneously as part of a method for selecting a biocatalyst according to the present invention, so that parallel to a test series of candidate biocatalysts a series of candidate substrates is tested, for instance by testing multiple (groups of) substrates for each candidate biocatalyst.

As stated above, the biocatalyst detection system as outlined herein may also consist of multiple interacting cells. For instance, two cell may interact when they produce certain compounds wherein each cell carries out one step in a synthesis reaction, the one utilizing the substrate as referred to herein and forming an intermediate compound which in turn functions as a substrate for the other cell that performs a subsequent bioconversion reaction. Intricate pathways for the formation of desired industrial products may thus be constructed. It should be understood that as the biocatalyst expression system may also comprise various conversion reactions from the substrate to the ultimate product that induces the product-inducible expression system with the detector gene, (i.e. the multi-step process as referred to herein) these multi-step bioconversions may be performed by two or more interacting host cells. In this way a chain of biocatalytic reactions may be constructed that can ultimately result in the advantageous production of sought-after chemicals. Therefore, the present invention in providing a method for detecting a biocatalyst, also provides a method for producing chemical compounds using the systems as disclosed herein.

### Outline of a preferred embodiment: selection process

The following provides further detail on the workings of the detection and selection approach as described herein. A method for detecting a biocatalyst according to the invention may essentially be performed as follows:

A multitude of host cells is provided, each comprising an expression system for a candidate biocatalyst and each provided with a product-inducible selector expression system as described herein, in this case with a product-inducible antibiotic resistance gene.

The host cells are introduced in a growth medium, comprising essential growth nutrients, as well as a test substrate and an antibiotic compound at a level sufficient to inhibit the growth of cells not capable of expressing the antibiotic resistance gene. The test substrate is a suitable substrate for the reaction which substrate can be converted into one or more products detected by the sensing component of the product-inducible reporter or selector system. In fact, a library of potential substrate compounds may be added to the medium, preferably in the form of defined groups, so that one or more suitable substrates can be detected and/or selected. The growth medium may further comprise one or more inducer compounds for initiating the expression of one or more biocatalysts inside the host cells in case their expression is inducible. The induction of one or more biocatalyst expression systems need not be continuous, but may also be discontinuous to the extent that expression is needed.

The host cell is allowed to express the candidate biocatalyst and the substrate is allowed to contact the biocatalyst. Upon said contact a period of time is allowed to lapse that is sufficient for conversion of the substrate into product and induction of the selector expression system by the product in cells that express a suitable biocatalyst. Upon expression of the selector expression system, cells that express a suitable biocatalyst will start to overcome the antibiotic growth inhibition and grow, while growth of the remaining cells will remain inhibited by the antibiotic compound.

The composition of the growth medium is determined by the host cell requirements. The concentration of substrate molecules in the growth medium will generally range between 1 µM and 1M. Suitable concentrations can easily be determined by the skilled person. Unless specified to the contrary, the reactions described herein preferably take place at atmospheric pressure over a temperature range from about 0 °C to about 150 °C, more preferably from about 5 °C to about 70 °C. The methods involving the host cells will generally be performed at a host cell-specific temperature, which in the case of micro-organisms may be in the range of about 20 °C-37 °C.

The host cell in aspects of the present invention recognizes the product chemical by using a specific chemosensor. A suitable example of such a chemosensor is a regulatory protein. Upon recognition or sensing of a given chemical, these chemosensors activate the transcription of a reporter or selector gene. In the example of the regulatory protein, the presence of a specific product of a chemical conversion reaction activates downstream transcription from a promoter-region and subsequent translation of the functional mRNA into the gene product of the reporter or selector gene. This general system is used herein to specifically sense a preselected biocatalysis product, and allows for the possibility of testing for the presence of biocatalysts that convert a substrate to the sensed product, for instance the conversion of the substrate benzaldehyde to the product benzoate by the biocatalyst benzaldehyde dehydrogenase.

The selection system based on product-dependent activation of transcription of a detector gene by a regulatory protein as described here above is shown in figure 1, which essentially depicts an illustrative example of the present invention.

Other aspects of the invention are a host cell for use in a method of the present invention, essentially as described in detail above.

Still another aspect of the present invention is a method of selecting a substrate for a pre-selected and defined biocatalytic reaction. For a detailed description of such a method explicit reference is made to the embodiments of the method of selecting a (bio)chemical compound as described above, which method is essentially similar, provided that instead of working with a population of candidate biocatalysts, the method is performed with a population of candidate substrates and by using essentially a host cell comprising a single functional biocatalyst expression system or a set of such biocatalyst expression systems coupled to a product-inducible marker expression system, which host cell is contacted with candidate substrates. As described above, testing compounds in pools has several advantages. Pooling can bring very large benefits in terms of cost and time efficiency, but at the cost of complexity. The chemical libraries of pharmaceutical companies are of the order of millions of compounds. It would take months to screen each compound individually against a particular biological target. Testing them in groups of 10 significantly decreases the time needed to screen the entire library. Very often, only a small fraction of the compounds in a chemical collection are active with respect to the biological target assayed. Consequently, when testing the compounds in groups, only a small number of pools will contain an active compound, which in turn means that only relatively few pools will test active and need to be decoded. The skilled person will understand that the same accounts for pooling candidate substrates, which is also envisioned in the present invention.

Yet another aspect of the present invention is a method of producing a (bio)chemical compound. Such a method comprises the selection of a biocatalyst by a method of the invention. Once the biocatalyst is selected, it may be isolated and optionally purified as described. Alternatively, the gene sequence can be unravelled so that the enzyme can be produced by transgenic methods well known in the art. Commonly, the host cell wherein the biocatalyst is produced at high levels is termed a production strain. The production strain may produce the biocatalyst intracellularly or may secrete the protein, optionally as a fusion protein. The protein can be isolated from the growth medium, optionally after removal of the cells. Additional processing, such as purification of the protein may be necessary. The skilled person is well acquainted with the various techniques for protein purification aimed at providing functional enzyme.

Once the biocatalyst is available (optionally in pure form or in the form of an organism comprising it), it may be used in a method for the manufacture of the product, simply by allowing it to convert the substrate under suitable reaction conditions. Conversion generally will take place in a reaction mixture comprising the biocatalyst and one or more substrates and if needed, cofactors, for the biocatalytic conversion reaction, optionally in the presence of additional compounds, such as buffers, chelators, ions or reducing compounds supporting the proper conformational folding of the enzyme.

The biotransformation of substrate into product can be performed by using permeabilized cells, resting cells, growing cells, or a combination of these as biocatalysts. The biotransformation can be performed by using crude cell extracts as biocatalysts. The biocatalysts can be immobilized on or in a water insoluble carrier or support system. The biotransformation can be performed in aqueous medium. It can also be performed in multi-phasic media possibly comprising two or more of the following: a solid phase, an aqueous phase, an organic phase, or a gaseous phase. Depending on the characteristics and purity of the enzyme, a sufficient time is allowed for the biotransformation reaction to proceed, after which the product or products and remaining substrate or substrates and cofactors may be recovered from the reaction mixture. Reaction conditions generally depend on the temperature optimum of the enzymatic reaction. Upon recovery, the product, remaining substrates and cofactors may be further purified by methods known in the art.

### MORE DETAILED ILLUSTRATION OF THE INVENTION

Hereinbelow the development of a host suitable for use in the selection approach is described in more detail. This represents the embodiment wherein the selector gene (the detector gene) is an antibiotic resistance gene, and thus, wherein the process is essentially based on selection for growth in the presence of an extracellularly added antibiotic.

As an illustration of the general principle, the host is tailored to sense the production of benzoate or 2-hydroxybenzoate from their corresponding aldehydes with an appropriate dehydrogenase, XylC from *Pseudomonas putida.* Clearly, the chemosensor system is expected to recognize these same compounds (benzoate or 2-hydroxybenzoate), as well as other compounds that happen to bind to the chemosensor in question, independently of the source of these compounds. They could be formed from other possible substrates via one or several biocatalytic reactions that the host cell is capable of carrying out, or via various metabolic pathways consisting of multiple biocatalysts present in the host cell, all of these reactions and pathways based on biocatalysts that are encoded on chromosomal or episomal DNA present in the host cell, or additional DNA introduced into the host cells. Any or several of these biocatalytic activities may have been present in the host cells or may have been introduced into the host cells knowingly or inadvertently. In all of these cases, if compounds that can interact with the sensing system are present or formed in the host cells or in modified (recombinant) host cells, the presence of these compounds will be sensed, resulting in a response triggered by the expression of the encoded detector genes. Given the different potential sources of compounds that can interact with the sensing system, resulting in detection or selection of cells that are presumed to contain the sought for biocatalyst but in fact contain or produce the same biocatalytic products formed via other means, this leads to so-called false positives, that is detection or selection of cells that appear to produce the sought for biocatalyst, but in fact contain alternative biocatalysts for the production of the compounds that interact with the sensing system. Various approaches are available to minimize the occurrence of false positives. One frequently used approach is to examine host cells that contain a complete product sensing system before they are further modified by the introduction of DNA or RNA sequences that contain possible biocatalyst encoding genes, to confirm that the sensing system of the host cells fails to recognize any of the products that can be sensed by the sensing system. Another frequently used approach is to examine the same host cells that contain a complete product sensing system before they are further modified by the introduction of DNA or RNA sequences that contain possible biocatalyst encoding genes, to confirm that the sensing system of the host cells fails to recognize any of the products that can be sensed by the sensing system when the substrate or substrates to be used in the identification and selection of desired biocatalysts are added to such cells.

Such cells, host cells that contain a sensing system, but no desired biocatalysts, should not recognize the presence of any one or several of the products that can be recognized by the sensing system. If products appear to be sensed, one or several test system modifications can be carried out until the modified detection or selection system containing host cells no longer produce product in the absence of added genes that encode the desired biocatalyst or biocatalysts. Test system modifications include the use of alternative host cells that are again used for the introduction of the sensing and detection or selection systems. Other modifications include altering the host cell to remove or reduce the effectiveness of genes that might encode biocatalysts that produce product that can be sensed by the sensing system of these host cells. Furthermore, it is possible to modify the test conditions such that the response of the host cell sensing system is reduced, for instance by introducing a higher antibiotic concentration in the growth selection experiments. Alternatively, the medium may be provided with substances capable of degrading excreted product. The net effect of these and other host cell or assay modifications is to reduce the response of the host cells in the absence of added substrates or added biocatalysts and preferably also added product, so that the effects of added substrates and added biocatalysts will be distinguishable from the effects of omitting substrates or biocatalysts, and so that production of product does not "leak through" to non-producing cells.

When the chemical conversion reaction involving the production of benzoate and 2-hydroxybenzoate from their corresponding aldehydes commences as a result of the intracellular presence of XylC (or any other appropriate dehydrogenase present in a host cell that contains DNA to encode such enzymes), the formed benzoate or 2-hydroxybenzoate is sensed by a regulatory sensing protein. This could be one of many proteins that bind small molecules, such as receptor proteins or antibodies. In the examples described here, a transcriptional regulatory protein is used that recognizes the product P, but not the substrate S, and then activates transcription of one of several possible detector genes (as illustrated in Figure 1). The chemosensor controls the expression of one or more detector genes, so that the presence of an active benzaldehyde dehydrogenase can be reported or, in a selective environment, be used to select for growth of only those cells that are biocatalytically active.

The selection approach is illustrated here for benzaldehyde dehydrogenase cloned in an *Escherichia coli* host that was modified to recognize the presence of one or more desired biocatalysis products P, but not the corresponding substrates S. The host strain was furthermore modified in such a manner that when the desired product P was recognized, this activated specific intracellular reporter expression systems or selection systems that enabled the cells to survive and grow under specific selective conditions. Cells which did not produce the desired product(s) P did not activate these reporter expression systems or selection systems. Such cells did not survive, and did not form colonies on selective plates (antibiotic comprising solid growth media). Thus, during growth on selective plates, only cells that produced the enzyme which in turn catalyzed the conversion of a substrate S to a desired product P were able to grow, and were hence selected.

The aspects as described herein can be used to detect a particular reaction product P by choosing a regulatory protein with the appropriate binding specificity. As a model reaction the oxidation of benzaldehyde or 2-hydroxybenzaldehyde to benzoate or 2-hydroxybenzoate by benzaldehyde dehydrogenase (XylC) from *Pseudomonas putida* was chosen. For the detection of benzoate or 2-hydroxybenzoate a previously described mutant of the transcriptional activator protein NahR from *P. putida* (Cebolla *et al.,* 1997) was chosen. This mutant recognizes both benzoic acids (the products), but not the corresponding aldehydes (the substrates), and activates transcription from its cognate salicylate promoter Pₛₐₗ. The system reports the presence of an active biocatalyst on the basis of either a screenable or a selectable cellular phenotype. The system easily selects biocatalyst containing cells present in the test culture at frequencies as low as 10⁻⁶.

As stated, the skilled person will appreciate that variations on this main theme are possible and will generally recognise a number of straightforward options. The biocatalyst detector system described here can be modified to recognize a wide range of biocatalysis products. This can be done first by exploiting the potential of a given regulator/promoter pair such as the NahR and Pₛₐₗ combination used here, and second, by developing other regulator/promoter pairs into suitable biocatalyst product chemosensors, followed by modifications of such new regulator/promoter pairs.

There is significant experience with both approaches. The effector profile of NahR has been altered (Cebolla *et al.,* 1997; Park *et al.,* 2005). NahR has the conserved domain architecture of a LysR type transcriptional regulator: a strongly conserved N-terminal domain with a HTH DNA binding motif and a C-terminal region required for multimerization are separated by a central region involved in effector recognition (Schell *et al.,* 1990; Tropel and van der Meer, 2004). Mutagenesis directed at the effector binding region produced mutants with higher sensitivity, lower basal detector gene expression, stronger induction and the ability to recognize non-natural effectors while retaining the ability to bind to DNA and activate transcription. Most importantly, however, NahR is far from being an exception. Examples of successful modifications of effector specificity can be found for members of almost all known bacterial transcriptional regulator families (see e.g. Ramos *et al.,* 1990; Ramos, *et al.,* 1986; Skarfstad *et al.,* 2000; Salto *et al.,* 1998; Devos *et al.,* 2002; Garmendia *et al.,* 2001; Wise *et al.,* 2000; Smirnova *et al.* 2004; Schumacher and Brennan 2002; Grkovic *et al.,* 2003; Scholz *et al.,* 2003; Henssler *et al.,* 2004; Bertram *et al.,* 2005). Early studies used random mutagenesis to isolate interesting mutants, as there was no structural information available (Schell *et al.,* 1990; Ramos *et al.,* 1990). When it became clear that transcriptional regulators possess conserved domain architectures, and functions such as DNA binding and effector recognition could be assigned to specific regions or domains it became possible to use directed mutagenesis, shuffling, domain swapping and structure based design (Skarfstad *et al.,* 2000; Salto *et al.,* 1998; Garmendia *et al.,* 2001; Smirnova *et al.* 2004; Scholz *et al.,* 2003; Henssler *et al.,* 2004; Hellinga *et al.,* 1998). All of the above approaches yielded regulators with altered effector profiles, which is consistent with the notion that structural information can be helpful, but is not required for directed evolution of proteins. Since the selection system depends only on the presence of product and not on how it was made, a single regulator can be used to monitor several enzymatic reactions. In the present case, the NahR mutant can detect nitrilase, amidase, aldehyde oxidase and aldehyde dehydrogenase activities yielding differently substituted benzoates.

In addition to the large existing body of knowledge about transcriptional regulators, several systematic and high-throughput approaches are available that should accelerate both the discovery of suitable regulators and the development of detector system plasmids. Discovery of new regulators is today possible *in vivo* and *in silico.* By using either a promoterless gfp-expression vector or a promoterless kanamycin resistance encoding transposon and high-throughput screening or selection, several investigators have been able to rapidly isolate regulators from bacterial genomes that responded to effectors of their choosing (Uchiyama *et al.,* 2005; Noda *et al.,* 2003). The conserved domain architecture, the typical small molecule binding domains and strongly conserved DNA binding domains serve as markers to retrieve potential regulators from genomic sequence databases (Perez-Rueda *et al.,* 2000; Anantharaman *et al.,* 2001; Babu *et al.,* 2003). Incorporation of newly identified regulator and promoter sequences into commercially available standardized detector plasmids can further reduce development time.

### Performance of the biocatalyst detector system

The present invention provides an in vivo biocatalyst detection strategy based on product recognition by a bacterial regulatory protein coupled to expression of a selection gene such as *tetA,* which permits growth of active biocatalyst containing selection hosts against a large background of inactive recombinants. We observed no false positives, indicating that this system is selective enough to discriminate between the product and substrate of an enzymatic reaction and between active and inactive enzymes. The sensitivity of the system was quite high, with detection of benzoate and benzaldehyde medium concentrations in the micromolar range. This allows sampling of reactions where substrates or products are toxic (Held *et al.* 1999) or poorly soluble in water (Cull *et al.,* 2001; Schmid *et al.,* 1998), problems that are often encountered in biocatalytic reactions of apolar compounds. Growth on selective media required higher concentrations of benzoate than benzaldehyde, probably because benzaldehyde entered the cells more readily than did benzoate.

In a first round of experiments it was observed that not all recombinants were able to withstand the selective conditions equally well. When the experiments were repeated with a host that displayed higher tetracycline tolerance, we obtained homogeneous growth at concentrations of around 250 uM benzoate or 100 uM benzaldehyde. When lower benzoate or benzaldehyde concentrations were used, we found that fewer cells grew under selective conditions. We conclude from this that even in a population of presumably isogenic recombinants there are substantial differences in fitness under selective pressure. It was always possible however to find an optimal window for growth by adapting the experimental conditions, allowing the selection of recombinants containing active enzymes while keeping background growth and false positives to a minimum.

### Perspectives of biocatalyst selectors

It is expected that the biocatalyst detector system described herein can easily be modified to recognize a wide range of biocatalysis products by replacing NahR and Pₛₐₗ with other regulator/promoter pairs. Once this is done for a specific substrate-product pair, both screening (via *lacZa* or other easily detectable markers) and selection (with *tetA* or other antibiotic resistance genes) for relevant biocatalysts becomes possible. The charm of the detection approach is that it requires only classical and very simple microbiological methods, such as culturing entire metagenome libraries (10⁷ - 10¹⁰ cells) in 1 - 100 ml liquid cultures and enriching for only those cells that survive antibiotics and presumably contain the biocatalysts of choice, thus enabling rapid selection of improved biocatalysts among shuffled or otherwise modified variants. In those cases where screening is preferred for the isolation of biocatalysts, additional flexibility can be achieved with reporter or selector genes coding for fluorescent proteins (Cormack *et al.,* 1996) or cell surface displayed binding proteins (Samuelson *et al.,* 2002), producing chemosensor systems compatible with powerful and established analytical methods like fluorescence activated cell sorting (FACS) (Daugherty *et al.,* 2000) and affinity chromatography (Patel *et al*., 2001; Ferenci and Lee, 1982).

In principle, it should be possible to select enzymes for any biocatalytic task, provided a regulatory protein with an appropriate product binding specificity is available, as suggested in Figure 1. The number of regulatory proteins known in bacteria is large (Stover *et al.,* 2000) for example *E. coli* K-12 and *P. aeruginosa* PAO1 each possess several hundred such proteins (Perez-Rueda *et al.,* 2000; Stover *et al.,* 2000), and many of these regulators recognize multiple compounds (Cebolla *et al.,* 1997; Shingler and Moore, 1994). In addition, each of the natural regulatory proteins can be modified by mutagenesis protocols (Cebolla *et al.,* 1997; Ramos *et al.,* 1990; Garmendia *et al.,* 2001) or computational design (Looger *et al.,* 2003; Hellinga, and Marvin 1998), to generate proteins with altered inducer or in this case, product specificities.

The selectivity of these sensing systems will enable the detection of a vast range of compounds, including their different regio- and stereoisomers, providing a generalized approach for the identification of desired product - biocatalyst combinations and for the subsequent selection of biocatalyst variants with improved activity and regio- and stereospecificity for a desired reaction.

The invention is now further illustrated in the following non-limiting examples.

### EXAMPLES

### Methods

Restriction enzymes, T4 DNA Ligase and Klenow DNA Polymerase were purchased from New England Biolabs Inc., Ipswich, MA, USA and Fermentas GmbH, St. Leon-Rot, Germany. Taq DNA Polymerase was from Sigma-Aldrich Corp. St. Louis, MO, USA and Dynazyme DNA Polymerase from Finnzyme, Espoo, Finland. All oligos for PCR amplification were custom synthetized by Microsynth AG, Balgach, Switzerland. The TOPO TA cloning Kit was obtained from Invitrogen, Breda, The Netherlands. All other chemicals were purchased either from Merck & Co, Whitehouse Station, NJ, USA, Fluka AG, Buchs SG, Switzerland or GERBU Biochemicals GmbH, Gaiberg, Germany.

### Strains, Plasmids, Media and General Procedures

The bacterial strains and plasmids used in this work are listed in Table 1. *Escherichia coli* strain DH10B was used for routine cloning, plasmid propagation and β-galactosidase or growth assays. Bacteria were grown in Luria-Bertani (LB) medium and supplemented where required with 100 µg/ml ampicillin, 25 µg/ml kanamycin, 30 µg/ml chloramphenicol and 15-25 µg/ml tetracycline. PCRs were carried out with Dynazyme DNA Polymerase using a Perkin-Elmer GeneAmp PCR system 9600 (Perkin-Elmer Inc., Wellesley, MA, USA). Subcloning of amplified fragments was done according to published protocols or with the TOPO TA cloning Kit following the manufacturers' protocol. Plasmid DNA was isolated with the Roche High Pure Plasmid Isolation Kit (Roche Diagnostics, F. Hoffmann-La Roche Ltd. Diagnostics Division, Basel Switzerland). All cloned PCR fragments were sequenced on a Li-Cor 4000L sequencer (LI-COR Biosciences GmbH, Bad Homburg, Germany) using the Amersham Thermosequenase cycle sequencing kit (Amersham Biosciences, Piscataway, NJ, USA) and IRD800-labelled primers (MWG-Biotech AG, Ebersberg, Germany).

### Construction of plasmids pVSF2-tetr and pVSF2-lacZ.

Plasmid pVSF2 was constructed from pMG209 and pCK01. Plasmid pMG209 is a dual-promoter plasmid with a salicylate promoter (P*ₛₐₗ*) and a T7 RNA polymerase promoter (PT7).

In addition, it contains the divergently transcribed *nahR* gene coding for the wild type transcriptional regulator NahR. Plasmid pCK01 (Fernandez *et al.,* 1995) is a low-copy cloning vector that confers chloramphenicol resistance. The mutation N169D and a *Pst*I restriction site were introduced into the *nahR* gene of pMG209 with the Quick Change mutagenesis Kit (Stratagene, La Jolla, CA), using the forward and reverse primers PnahRmtl (5'-gccggctgct**g**ca**g**gatcactacgtgtgcc-3', mutagenized bases bold, *Pst*I site underlined) and PnahRmt2 (5'-ggcacacgtagtgat**c**ctg**c**agcagccggc-3'), respectively. Successfully mutagenized plasmids were identified by restriction with *Pst*I and used as template for PCR amplification with primers PHindpMG (5'-cccaagcttaattgtaatccggatatagttcctcctttcagc-3') and PEcopMG (5'-ccggaattcggtgcatgctagatcaatccgtaaacagg-3') to yield a 2176 bp fragment containing the mutagenized *nahR* gene, the salicylate and T7 promoter, a 771 bp stuffer fragment and a T7 terminator. The PCR fragment was cloned into the blue/white selection vector pCR2.1-TOPO and sequenced with primers -40fw (5'-agggttttcccagtcacgacgtt-3') and -40rv (5'-gagcggataacaatttcacacagg-3'). To re-isolate the fragment the resulting pCR2.1-TOPO-construct was linearized with *Not*I, blunted with Klenow DNA polymerase and digested with *BamH*I to yield a 2260 bp fragment with one blunted and one overhanging end. Plasmid pCK01 was linearized with *Nde*I, blunted with Klenow DNA polymerase and digested with *BamH*I to yield a 3097 bp fragment with one blunted and one overhanging end containing the chloramphenicol resistance gene, the origin of replication and genes required for plasmid replication. Ligation of these two fragments yielded the low-copy plasmid pVSF2.

To construct pVSF2-lacZ, *LacZa* was generated on template pCK01 with primers PlacZfw (5'-cgccatatgaccatgattacgaattgcggc-3') and PlacZrv (5'-cctcgagttagcgccattcgccattcagg-3'). The 267 bp fragment was cloned into pCR2.1-TOPO and sequenced with primers -40fw and -40rv. The fragment was excised from pCR2.1-TOPO with *Nde*I/*Xho*I and ligated into pVSF2.

To construct pVSF2-tet^{r}, *tetA* was amplified from pUC26 with primers PtetNde (5'-ggaattccatatgaaatctaacaatgcgctcatcgtcatcc-3') and PtetXho (5'-ccgctcgagtcaggtcgaggtggcccggctcc-3'). The 1210 bp fragment was cloned into pCR2.1-TOPO and sequenced with primers -40fw and -40rv. The fragment was excised from pCR2.1-TOPO with *Nde*I/*Xho*I and ligated into pVSF2.

### Plasmids pCom10 and pCom10-XylC

XylC was placed under the control of the Pₐₗₖ promoter in pCom10. Plasmid pCom10 is a high-copy expression vector based on the alkane-responsive promoter P_{alkB} and its positive regulator AlkS, that is compatible with the low-copy reporter plasmids. Expression of XylC from the Alk promoter is regulated by the transcriptional activator AlkS and can be induced by decane. To construct pCom10-XylC, the *xylC* gene coding for benzaldehyde dehydrogenase XylC from *Pseudomonas putida* was amplified from pCK04AXylR with primers PXylCfw (5'-cgccatatgcgggaaacaaaagagcagcc-3') and PXylCrv (5'-caagctttcaaaatgggtaattagctggcttctca-3'). The resulting 1477 bp fragment was cloned into pCR2.1-TOPO, sequenced with primers -40fw and -40rv, excised from pCR2.1-TOPO with *Nde*I/*Hind*III and ligated into pCom10.

### Curing DH10B-pVSF2-tetr/pCom10-XylC of pCom10-XylC.

DH10B-pVSF-tet^{r}/pCom10-XylC was cured of pCom10-XylC by three successive rounds of growth in liquid LB medium containing only chloramphenicol (30 µg/ml) and 0.05 % dicyclopropylketone (DCPK). Since plasmid pCom10-XylC confers kanamycin resistance, kanamycin was omitted from the medium. Addition of DCPK induced expression of *xylC* and resulted in selection pressure against cells containing pCom10-XylC. After three rounds, cells were analyzed on LB/agar plates containing either only chloramphenicol (30 µg/ml) or chloramphenicol and kanamycin (25 µg/ml). Plasmids were isolated from cells that were able to grow on plates containing chloramphenicol but had lost the ability to grow in the presence of kanamycin. Restriction analysis of plasmids confirmed that cells were cured of pCom10-XylC and retained pVSF2-tet^{r}.

### Plate-Based Assay for β-Galactosidase Activity

40 µl of a 2 % X-Gal solution in DMF was spread on LB/agar plates containing chloramphenicol (30 µg/ml) and varying concentrations of benzoate or 2-hydroxybenzoate. *E. coli* DH10B cells harbouring reporter plasmid pVSF2-lacZ were streaked on the plates from a liquid culture that had been grown at 30 °C to an OD₆₀₀ of 0.1. Cells were grown for 36 h at 30 °C followed by incubation at 4 °C for 24 h after which blue colony color formation was evaluated visually.

*E. coli* DH10B cells harbouring the reporter plasmid pVSF2-lacZ together with either the catalyst containing plasmid pCom10-XylC or, as a control, the empty pCom10 vector, grown in a liquid culture at 30 °C to an OD600 of 0.1, were similarly streaked on identical X-Gal/chloramphenicol plates supplemented with kanamycin (25 µg/ml) and various concentrations of either benzaldehyde or 2-hydroxybenzaldehyde. Cells were plated and grown as above, under decane vapor, followed by incubation at 4 °C and testing for blue colony color as above.

### Plate-Based Assay for Tetracycline Resistance

Approximately 10³ *E. coli* DH10B cells, harbouring the reporter plasmid pVSF2-tet^{r}, grown in liquid culture at 30 °C to an OD₆₀₀ of 0.1, were spread onto non-selective LB/agar plates containing only chloramphenicol (30 µg/ml) and onto selective plates containing chloramphenicol (30 µg/ml), tetracycline (22.5 µg/ml) and varying concentrations of benzoate. Identical non-selective and selective plates were supplemented with varying concentrations of 2-hydroxybenzoate, except that the tetracycline concentration was 25 µg/ml. Plates were incubated for 36 h at 30 °C. Each plate assay was conducted in triplicate and growth was determined by measuring the colony diameter of 10 solitary colonies for each plate.

Approximately 10³ *E. coli* DH10B cells harbouring the reporter plasmid pVSF2-tet^{r} and either the catalyst containing plasmid pCom10-XylC or, as a control, the empty pCom10 vector, were similarly grown and spread onto non-selective LB/agar plates containing only chloramphenicol (30 µg/ml) and kanamycin (25 µg/ml) and onto selective plates containing chloramphenicol (30 µg/ml), kanamycin (25 µg/ml), tetracycline (22.5 µg/ml) and varying concentrations of benzaldehyde. Plates were incubated for 36 h at 30 °C under decane vapor, and colony size was assessed in triplicate as above. The assay was similarly performed with various concentrations of 2-hydroxybenzaldehyde, except that the tetracycline concentration was 25 µg/ml.

### Plate-Based Selection for a Functional Enzyme

Strain DH10B harbouring pVSF2-tet^{r} and either pCom10 or pCom10-XylC was grown in liquid culture to an OD600 of 0.1. The two cultures were diluted and mixed in ratios of 10⁴:1, 10⁵:1 and 10⁶:1, keeping the amount of cells containing pCom10-XylC constant at 10² while increasing the amount of cells containing pCom10 from 10⁶ to 10⁷ to 10⁸. The cells were spread onto selective LB/agar plates containing chloramphenicol (30 µg/ml), kanamycin (25 µg/ml), tetracycline (25 µg/ml) and either benzaldehyde (200 µM) or 2-hydroxybenzaldehyde (5 µM) and incubated for 36 h at 30 °C during which time the cells were exposed to decane vapor. For each ratio and substrate the plate assay was conducted in triplicate and per ratio and substrate the plasmids of 10 colonies were isolated and analyzed by restriction digest with *Nde*I and *Hind*III for the presence of the *xylC* gene. In addition, the 60 colonies were subjected to a second assay. The cells were re-streaked onto selective LB/agar plates containing chloramphenicol (30 µg/ml), kanamycin (25 µg/ml), tetracycline (25 µg/ml) and either no substrate, 200 µM benzaldehyde or 5 µM 2-hydroxybenzaldehyde. Plates were incubated for 36 h at 30 °C under decane vapor.

Two plasmids were constructed with different reporter or selector genes fused to the salicylate promoter. Plasmid pVSF2-lacZ carries a gene coding for the α-fragment of 6-galactosidase, while plasmid pVSF2-tet^{r} carries the tetracycline resistance gene *tetA.* Both plasmids carry a constitutively expressed gene coding for a mutated form of the regulatory protein NahR (figure 2). In contrast to wild type NahR, this mutant recognizes benzoate as an inducer and has an increased affinity for the natural inducer 2-hydroxybenzoate. *E. coli* hosts that carry either plasmid should express the reporter or selector genes, producing a screenable (*lacZa*) or a selectable (*tetA*) phenotype when grown in the presence of benzoate and 2-hydroxybenzoate.

### Screening for functional biocatalysts based on β-galactosidase

To show that expression of the lacZa reporter or selector gene was induced by benzoate and 2-hydroxybenzoate, plasmid pVSF2-lacZ was introduced into *E. coli* DH10B. The response of the resulting benzoate reporter strain was determined by streaking recombinants on plates containing chloramphenicol, X-Gal and varying concentrations of benzoate or 2-hydroxybenzoate (Table 2 and 3). Blue colonies of DH10B-pVSF2-lacZ were detected at benzoate concentrations higher than 100 µM and 2-hydroxybenzoate concentrations higher than 10 µM, with a saturating concentration around 500 µM or 100 µM 2-hydroxybenzoate, respectively. Blue colonies were not formed in the absence of inducers.

To demonstrate that benzoate and 2-hydroxybenzoate formed enzymatically from the corresponding benzaldehydes could also induce expression of the reporter or selector gene we introduced benzaldehyde dehydrogenase (XylC) into the strain. Strain DH10B-pVSF2-lacZ was transformed with pCom10-XylC, streaked on plates containing chloramphenicol, kanamycin, X-Gal and varying concentrations of benzaldehyde or 2-hydroxybenzaldehyde, and grown exposed to decane vapor to induce XylC. Strain DH10B-pVSF2-lacZ, carrying an empty pCom10 vector served as control. Tables 4 and 5 show that on plates containing the XylC substrates benzaldehyde (Table 4) or 2-hydroxybenzaldehyde (Table 5), the biocat - reporter strain (DH10B-pVSF2-lacZ/pCom10-XylC) formed blue colonies at benzaldehyde concentrations above 50 µM and 2-hydroxybenzaldehyde concentrations above 25 µM. In the absence of substrate, the colonies remained white. The control strain (DH10B-pVSF2-lacZ/pCom10) showed no blue colonies on addition of benzaldehydes.

### Development of a biocatalyst - selector host

Having shown that catalysis can be coupled to reporter or selector gene expression, we replaced *lacZa* with *tetA* to go from a β-galactosidase based screen to a tetracycline resistance based selection system. In preliminary experiments with *E. coli* DH10B harbouring pVSF2-tet^{r} we observed benzoate-dependent growth on selective plates containing varying concentrations of benzoate. However, only about 0.1 % of plated cells were able to grow under these conditions. Of the colonies that appeared on the plates some were able to grow faster than others, resulting in an inhomogeneous population of small and large colonies. When DH10B-pVSF2-tet^{r} was transformed with pCom10-XylC we observed benzaldehyde-dependent growth, again of only about 0.1 % of plated cells, forming small and large colonies. We repeated the plate assay with several of these small and large colonies. Cells that previously formed small colonies performed very poorly in the plate assay, with only about 0.01-0.05 % of plated cells able to grow. However, cells picked from large colonies showed the desired behavior. All cells plated were able to grow and formed similar sized colonies. When tetracycline was omitted from the assay no difference could be seen between the small and large colony forming cells.

Some cells displayed a higher tetracycline tolerance that is independent of XylC since both DH10B-pVSF2-tet^{r} with and without pCom10-XylC showed this effect. To investigate whether this higher tetracycline tolerance was caused by a mutation in the host or on the reporter plasmid pVSF2-tet^{r} we extracted pVSF2-tet^{r} from one slow and one fast growing DH10B-pVSF2-tet^{r}/pCom10-XyIC strain. Fresh DH10B transformants, transformed with these different pVSF2-tet^{r} plasmids, grew equally well, indicating that the higher tetracycline tolerance of the fast growing DH10B-pVSF2-tet^{r}/pCom10-XylC strain was related to a mutation in the host rather than the reporter plasmid.

The fast growing DH10B-pVSF2-tetr/pCom10-XylC strain was then cured of plasmid pCom10-XylC. The resulting DH10B-pVSF2-tet^{r} strain was able to grow on non-selective plates without inducers, whereas growth on selective plates required addition of benzoate or 2-hydroxybenzoate.

The effect of benzoate and 2-hydroxybenzoate on growth is dose dependent (figure 3): all cells plated were able to grow on plates with benzoate concentrations above 500 µM or 2-hydroxybenzoate concentrations above 15 µM. On plates containing 250 µM benzoate or 10 µM 2-hydroxybenzoate, however, we observed not only smaller but also fewer colonies. This provided us with a suitable biocatalyst selection host.

### Response of the selector host to XylC substrates

Plasmid pCom10-XylC was re-introduced into DH10B-pVSF2-tet^{r} to produce the desired biocat - selector strain DH10B-pVSF2-tet^{r} / pCom10-XylC. This strain grew on tetracycline containing selective plates only after addition of benzaldehyde or 2-hydroxybenzaldehyde.

As previously observed with the benzoic acids, the effect of aldehydes on growth also showed a dose dependence (figure 4). On plates containing more than 100 µM benzaldehyde or 2 µM 2-hydroxybenzaldehyde, all plated cells grew. As the aldehyde concentration was lowered, colony diameter and number also decreased. The control strain DH10B-pVSF2-tet^{r}/pCom10, which contains no biocatalyst, failed to grow on addition of aldehydes. As expected, both strains were able to grow on non-selective plates without aldehydes.

We compared the sensitivity of the selector host for substrates and products by plotting the data of Figures 3 and 4 as inverse plots of 1/[colony diameter] vs 1/[compound] and estimating the concentration required for half maximal colony size. About 400 µM benzoate and 20 µM 2-hydroxybenzoate were needed to produce colonies with diameters equal to 50% of the maximum diameters seen in the experiments of Figure 3. When cells containing XylC were plated with the corresponding substrates, about 6 µM benzaldehyde and 0.3 µM 2-hydroxybenzaldehyde were needed to produce colonies with half-maximal diameters (Figure 4). Thus, the aldehyde substrates were about 65 times more effective than the acid products in triggering the antibiotic resistance response and the 2-hydroxy substituted compounds were about 15 - 20 fold more effective than the parent compounds in triggering a response.

### Selection of functional biocatalysts with the benzoate selection host

Our goal was to enable the selection of cells that contain active enzymes from a large population background containing no or less active enzymes. To demonstrate that this is possible with our selection system, DH10B cells containing the active enzyme XylC were mixed with cells lacking the enzyme in ratios of 1:10⁴, 1:10⁵ and 1:10⁶ and plated on selective and non-selective plates.

The number of colonies that appeared on selective plates containing benzaldehyde or 2-hydroxybenzaldehyde corresponded to the number of enzyme containing cells plated. For each ratio and substrate, 10 colonies were tested for pCom10-XylC.

We found 100 % positives for ratios of 1:10⁴ and 1:10⁵ for both substrates (figure 5), and 90 % (benzaldehyde) or 70 % (2-hydroxybenzaldehyde) positives for a ratio of 1:10⁶. In addition, the 60 picked colonies were subjected to a second assay to verify that they did indeed show aldehyde-dependent growth. All candidates confirmed as positives were able to grow in the presence of aldehydes and failed to grow in the absence of aldehydes. The four false positives found on plates with 10⁻⁶ positive/inactive cells grew both in the presence and absence of aldehydes and could thus be singled out by this secondary selection round.

The selection of biocatalyst containing cells in a mixture of a large number of cells that do not contain biocatalyst was illustrated for the enzyme benzaldehyde dehydrogenase that converts benzaldehyde and related compounds (e.g., 2-hydroxybenzaldehyde) to benzoate or the corresponding related acids (e.g., 2-hydroxybenzoate). When benzaldehyde or related aldehydes were converted to products (benzoate or related acids), these products could bind to a receptor protein encoded by a *nahR** variant that has been introduced into the host cell; the receptor protein is a transcriptional activator which can activate one or more of several proteins or enzymes that together produce a detectable signal (e.g., color) or permit growth in the presence of an antibiotic. Thus, in this example we examined benzoate production via the transcriptional activator protein NahR*, coupling product recognition to expression of *lacZa,* a color indicator, or *tetA,* an antibiotic resistance gene. During incubation on tetracycline containing plates, cells that did not produce benzoate failed to grow, while cells that contained active enzyme grew, permitting their selection from a large population of inactive variants that did not grow in the presence of the antibiotic. Such systems signal the conversion of substrate to product, and hence indicate that the cells contain an enzyme with the required biocatalytic activity.

### The biocatalyst selector system

Colonies developed when test cells were plated on selective media containing benzoic or 2-hydroxybenzoic acid. These XylC products bound the NahR regulator, and induced *tetA* expression with consequent tetracycline antiporter activity, enabling cell growth in the presence of tetracycline. Colonies were formed with minimum concentrations of 10 µM 2-hydroxybenzoate or 100 µM benzoate. Cells plated on selective media containing the substrates benzaldehyde or 2-hydroxybenzaldehyde formed colonies only when they expressed *xylC* and produced the corresponding products.

### Differential effects of extracellularly added substrates and products

Interestingly, the substrate concentrations required to produce half-maximal colony diameters were 65-fold lower than the corresponding product concentrations needed for the same effect. This can be considered to be due to differences in the transport of benzaldehyde and benzoate through the *E. coli* cell envelope. Such transport is believed to involve only passive diffusion ^{21,22}. Based on recent studies of the oxidation of toluene to benzoic acid by *E. coli* hosts containing the xylene oxidation system (*xylMA*) of *Pseudomonas putida* mt-2 ²³, benzaldehyde and benzoate diffusion rates across *E. coli* cell membranes can be estimated to exceed 180 and 10 µmol/min/g cell dry mass, respectively.

Thus, substrate influx and product efflux, though different, are quite rapid. Intracellular and extracellular benzaldehyde and benzoate concentrations equilibrate within a few seconds and one minute, respectively. Substrate is oxidized to benzoate by XylC, and accumulates to a steady state intracellular concentration dictated by the relative synthesis and efflux rates. Passive efflux increases as the intracellular benzoate concentration increases until the benzoate efflux rate equals the benzoate synthesis rate, resulting in a steady state benzoate concentration.

Thus, when benzaldehyde is added externally to 6 µM, the concentration needed for half maximal colony development (Figure 4), and oxidized intracellularly by XylC, the benzoate efflux rate can be expected to equal the benzoate synthesis rate when a steady state benzoate concentration of around 400 µM is reached, the concentration needed for half maximal colony development when added extracellularly (Figure 3). Given its relatively rapid equilibration under these conditions, benzoate is expected to also be present at this concentration intracellularly. Interestingly, the apparent affinity of benzoate for the NahR variant used here has been estimated at *K_{d}* = 0.5 mM by de Lorenzo and coworkers (Cebolla *et al.,* 1997), similar to the benzoate concentration necessary for half maximal colony development seen in Figure 3. This benzoate concentration, sensed by NahR, triggers sufficient tetracycline antiporter activity to enable half maximal colony development. At extracellular benzaldehyde concentrations below 6 µM, the rate of benzoate synthesis decreases. The corresponding lower efflux rates result in lower steady state benzoate concentrations, lower tetracycline antiporter activities, higher intracellular tetracycline concentrations, and smaller colony diameters, as seen in Figures 3 and 4. For benzaldehyde concentrations greater than 6 µM, benzoate will be synthesized faster, resulting in a higher steady state benzoate concentration, and larger colonies will be formed.

### Tuning the biocatalyst selection system

To tune the selection system, we first optimized the substrate concentration to yield a steady state product concentration that activated NahR only in those cells that contained biocatalytic activity. Second, the antibiotic concentration was chosen to enable selection of cells that contain biocatalyst activity. These conditions were established easily and quickly by plating a large population of biocatalyst containing cells with several substate and antibiotic concentrations.

The effect of the substrate concentration is illustrated by results obtained with the 2-hydroxy substituted substrate and product set. About 15 - 20 times less 2-hydroxybenzoate than benzoate was needed to trigger tetracycline resistance and enable growth of test cells. When substrate rather than product was added to cells that contained XylC, colonies developed to half maximal diameter at only 0.3 µM 2-hydroxybenzaldehyde, compared to the 20 µM 2-hydroxybenzoate needed for similar colony development. Thus, as was the case with the unsubstituted benzaldehyde and benzoate, the substrate was ca. 65 times more efficient than the product in triggering tetracycline resistance. Here also, the 2-hydroxy substituted substrate evidently crossed the cytoplasmic membrane more easily than did the corresponding product. However, since the affinity of the NahR chemosensor is 10 times greater for 2-hydroxybenzoate than for benzoate, as reported by de Lorenzo and coworkers (Cebolla *et al.,* 1997), correspondingly lower concentrations of the product and substrate were needed to trigger expression of *tetA* and growth on tetracycline containing selective plates. Thus, compared to 400 µM benzoate only 20 µM 2-hydroxybenzoate was sufficient to trigger a half-maximal colony diameter. It is possible to maintain such a low steady state product concentration with significantly lower 2-hydroxybenzoate synthesis and efflux rates. This could explain why the extracellular 2-hydroxybenzaldehyde concentration needed for half-maximal colony development was only 0.3 µM (Figure 4).

The system can also be modulated via the tetracycline concentration used for selection. Initial growth assays with benzoate were carried out with tetracycline concentrations of 22.5 µg/ml. When we attempted to select active biocatalyst containing hosts against large backgrounds of inactive hosts, we observed large numbers of false positives. We therefore increased the stringency by increasing the tetracycline concentration to 25 ug/ml and decreasing the substrate concentration, after which we observed 100 % positives at frequencies of 1 in 10⁴ and 1 in 10⁵ biocatalyst containing cells and 70 - 90% positives for frequencies of 1 in 10⁶.

The ability to fine-tune the stringency of a selection through altering basic parameters is important if one wishes to test a library for variants that show a 2-fold or smaller improvement in catalytic activity. If two enzymes with small differences in activity are tested under conditions where both signals (product concentrations) saturate the chemosensor protein, it is simplest to lower the concentration of the extracellularly added substrate, possibly in combination with a change of the tetracycline concentration, as illustrated by the 2-hydroxybenzaldehyde experiments,. More complex, but useful in biocatalyst optimization experiments, a reduction of catalyst expression levels can reduce the initial biocatalyst activity to a range that allows comparisons between closely related variants. This can be done by using low-copy or tightly regulatable expression vectors, weak or modified promoters or inefficient ribosome binding sites (Couturier *et al.,* 1988; Ayoubi and Van de Ven, 1996; de Boer and Hui, 1990).

### The dynamic range and resolution of the test system

Colony size is a function of the growth rate µ. This depends on the maximum growth rate µₘₐₓ and the steady state intracellular tetracycline concentration, which is a function of the medium tetracycline concentration, the rate of tetracycline influx, and the antiporter activity elicited by product binding to NahR in our experiments. We have used optimized extracellular tetracycline concentrations between 15 and 25 µg/ml , equivalent to 34 - 56 µM. These lead to intracellular concentrations which are then modulated by antiporter activity. Tetracycline binds to the ribosomal A site with a K_{d} that has been variously reported as 5 µM (Burdettt, 1996, Connell *et al.,* 2003), and up to 30 µM. An intracellular tetracycline concentration equal to K_{d} reduces ribosomal activity to half maximal levels, which, as long as there are no other major growth limiting factors, reduces the maximum growth rate µₘₐₓ seen in our plating experiments (µₘₐₓ = 0.5 to 0.52 h⁻¹) to µ = 0.25 - 0.26 h⁻¹.

Colony diameter is a sensitive measure of growth rate differentials. Based on typical *E. coli* dimensions of 1 µm³ per wet cell, 30% space between cells in the colony, and assuming no lag in cell division after plating, colonies with growth rates below 0.25 h⁻¹ contain at most 8,200 cells after 36 hours, and have diameters smaller than 0.05 mm. Such colonies are essentially invisible. The growth rates needed to produce the colonies seen in Figure 5, which develop diameters of 0.2 to 1.0 mm in 36 hours, range from µ = 0.39 h⁻¹ to 0.50 h⁻¹, or about 80 - 100% of µₘₐₓ. The intracellular tetracycline concentration, which must have been in excess of K_{d} for the biocatalytically inactive cells, must have been maintained substantially below 10% of K_{d} for 1 mm colonies that grew at µ = 0.50 h⁻¹, and reduced to ca. 35 - 40% of K_{d} for 0.2 mm colonies that grew at µ = 0.39 h⁻¹. Thus, a 1.29-fold range of growth rates resulting in a 5-fold range of colony diameters in our plating experiments reports on a range of intracellular tetracycline concentrations of ca. 5 - 50% of K_{d}.

We conclude that the system can be tuned effectively via first, optimization of the specific substrate concentration and the selective extracellular tetracycline concentration used, which enable changes of the dynamic range as needed, and second, via colony diameter comparisons, which provides significant amplification of small changes in tetracycline concentration and hence antiporter activity that results from product steady state concentrations, allowing distinctions between closely related biocatalyst activities within a given experiment.

### REFERENCES

Abril, M. A., C. Michan, et al. Regulator and enzyme specificities of the TOL plasmid-encoded upper pathway for degradation of aromatic hydrocarbons and expansion of the substrate range of the pathway. J. Bacteriol. 171 (12), 6782-90 (1989).
Amann, E., J. Brosius, et al. Vectors bearing a hybrid trp-lac promoter useful for regulated expression of cloned genes in Escherichia coli. Gene 25 (2-3), 167-178 (1983).
Anantharaman, V., Koonin, E. V. & Aravind, L. Regulatory potential, phyletic distribution and evolution of ancient, intracellular small-molecule-binding domains. J. Mol. Biol. 307, 1271-92 (2001).
Arvidson, D. N., M. Shapiro, et al. Mutant Tryptophan Aporepressors With Altered Specificities of Corepressor Recognition. Genetics 128 (1), 29-35 (1991).
Ayoubi, T. A. Y. & Van de Ven, W. J. M. Regulation of gene expression by alternative promoters. FASEB J. 10, 453-60 (1996).
Babu, M. M. & Teichmann, S. A. Evolution of transcription factors and the gene regulatory network in Escherichia coli. Nucleic Acids Res. 31, 1234-1244 (2003).
Bertram, R. et al. Phenotypes of Combined Tet Repressor Mutants for Effector and Operator Recognition and Allostery. J.Mol. Microbiol. and Biotechnol. 8, 104-110 (2005).
Buhler, B., A. Schmid, et al. Xylene monooxygenase catalyzes the multistep oxygenation of toluene and pseudocumene to corresponding alcohols, aldehydes, and acids in Escherichia coli JM101. Journal of Biological Chemistry 275 (14), 10085-10092 (2000).
Burdett, V. Tet(M)-promoted release of tetracycline from ribosomes is GTP dependent. J. Bacteriol. 178 (11), 3246-51 (1996).
Burton, S. G., Cowan, D. A. & Woodley, J. M. The search for the ideal biocatalyst. Nat Biotechnol 20, 37-45 (2002).
Cebolla, A., Sousa, C. & de Lorenzo, V. Effector specificity mutants of the transcriptional activator NahR of naphthalene degrading Pseudomonas define protein sites involved in binding of aromatic inducers. J. Biol. Chem. 272, 3986-3992 (1997).
Chevalet, L., A. Robert, et al. Recombinant protein production driven by the tryptophan promoter is tightly controlled in ICONE 200, a new genetically engineered E. coli mutant. Biotechnol. Bioeng. 69 (4), 351-8 (2000).
Connell, S. R., D. M. Tracz, et al. Ribosomal Protection Proteins and Their Mechanism of Tetracycline Resistance. Antimicrob. Agents Chemother. 47 (12), 3675-3681 (2003).
Cormack, B. P., Valdivia, R. H. & Falkow, S. FACS-optimized mutants of the green fluorescent protein (GFP). Gene 173, 33-38 (1996).
Couturier, M., Bex, F., Bergquist, P. L. & Maas, W. K. Identification and classification of bacterial plasmids. Microbiol. Rev. 52, 375-95. (1988).
Cull, S. G., Woodley, J. M. & Lye, G. J. Process selection and characterisation for the biocatalytic hydration of poorly water soluble aromatic dinitriles. Biocatal. Biotransform. 19, 131-154 (2001).
Daugherty, P. S., Iverson, B. L. & Georgiou, G. Flow cytometric screening of cell-based libraries. J. Immunol. Methods 243, 211-227 (2000).
Daunert, S., G. Barrett, et al. Genetically Engineered Whole-Cell Sensing Systems: Coupling Biological Recognition with Reporter Genes. Chem. Rev. 100 (7), 2705-2738 (2000).
de Boer, H. A. & Hui, A. S. Sequences within ribosome binding site affecting messenger RNA translatability and method to direct ribosomes to single messenger RNA species. Methods Enzymol. 185, 103-14. (1990).
Devos, D., Garmendia, J., de Lorenzo, V. & Valencia, A. Deciphering the action of aromatic effectors on the prokaryotic enhancer-binding protein XylR: a structural model of its N-terminal domain. Environ. Microbiol. 4, 29-41 (2002).
Ferenci, T. & Lee, K. S. Directed evolution of the lambda receptor of Escherichia coli through affinity chromatographic selection. J. Mol. Biol. 160, 431-44 (1982).
Fernandez, S., de Lorenzo, V. & Perez-Martin, J. Activation of the transcriptional regulator XylR of Pseudomonas putida by release of repression between functional domains. Mol. Microbiol. 16, 205-13 (1995).
Fieser, LF & Fieser, M. Reagents for Organic Synthesis; Wiley & Sons, New York, Volumes 1-15 (1968-)
Gabor, E. M., de Vries, E. J. & Janssen, D. B. Construction, characterization, and use of small-insert gene banks of DNA isolated from soil and enrichment cultures for the recovery of novel amidases. Environmental Microbiology 6, 948-958(2004).
Gamper, M., Hilvert, D. & Kast, P. Probing the Role of the C-Terminus of Bacillus subtilis Chorismate Mutase by a Novel Random Protein-Termination Strategy. Biochemistry 39, 14087-14094 (2000).
Garmendia, J., Devos, D., Valencia, A. & De Lorenzo, V. A la carte transcriptional regulators: unlocking responses of the prokaryotic enhancer-binding protein XylR to non-natural effectors. Mol. Microbiol. 42, 47-59 (2001).
Goddard, J.-P. & Reymond, J.-L. Enzyme assays for high-throughput screening. Curr. Opin. Biotechnol. 15, 314-322 (2004b).
Goddard, J.-P. & Reymond, J.-L. Recent advances in enzyme assays. Trends Biotechnol. 22, 363-370 (2004a).
Griffiths, J. S. et al. A bacterial selection for the directed evolution of pyruvate aldolases. Bioorg. Med. Chem. 12, 4067-4074 (2004).
Grkovic, S., Hardie, K. M., Brown, M. H. & Skurray, R. A. Interactions of the QacR multidrug-binding protein with structurally diverse ligands: implications for the evolution of the binding pocket. Biochemistry42, 15226-36 (2003).
Hakkila, K., M. Maksimow, et al. Reporter genes lucFF, luxCDABE, gfp, and dsred have different characteristics in whole-cell bacterial sensors. Anal. Biochem. 301 (2), 235-42 (2002).
Held, M. et al. An integrated process for the production of toxic catechols from toxic phenols based on a designer biocatalyst. Biotechnol. Bioeng. 62, 641-648 (1999).
Hellinga, H. W. & Marvin, J. S. Protein engineering and the development of generic biosensors. Trends Biotechnol. 16, 183-189 (1998).
Henssler, E.-M., Scholz, O., Lochner, S., Gmeiner, P. & Hillen, W. Structure-Based Design of Tet Repressor To Optimize a New Inducer Specificity. Biochemistry 43, 9512-9518 (2004).
Hu, M. C.-T. and N. Davidson. The inducible Iac operator-repressor system is functional in mammalian cells. Cell 48 (4), 555-566 (1987).
Kim, J.-Y., Choi, G.-S., Jung, I.-S., Ryu, Y.-W. & Kim, G.-J. A systematic approach for yielding a potential pool of enzymes: practical case for chiral resolution of (R,S)-ketoprofen ethyl ester. Protein Engineering 16, 357-364 (2003).
Looger, L. L., Dwyer, M. A., Smith, J. J. & Hellinga, H. W. Computational design of receptor and sensor proteins with novel functions. Nature 423, 185-190 (2003).
MacBeath, G., Kast, P. & Hilvert, D. Redesigning enzyme topology by directed evolution. Science 279, 1958-1961 (1998).
Noda, K., Watanabe, K. & Maruhashi, K. Cloning of a rhodococcal promoter using a transposon for dibenzothiophene biodesulfurization. Biotechnol. Lett. 25, 1875-82 (2003).
Noyori, R. Asymmetric Catalysis in Organic Synthesis. Wiley and Sons, New York (1994).
Park, H. H., Lee, H. Y., Lim, W. K. & Shin, H. J. NahR: effects of replacements at Asn 169 and Arg 248 on promoter binding and inducer recognition. Arch. Biochem. Biophys. 434, 67-74 (2005).
Patel, D. et al. Continuous affinity-based selection: rapid screening and simultaneous amplification of bacterial surface-display libraries. Biochem. J. 357, 779-785 (2001).
Perez-Rueda, E. & Collado-Vides, J. The repertoire of DNA-binding transcriptional regulators in Escherichia coli K-12. Nucleic Acids Res. 28, 1838-1847 (2000).
Ramos, J. L., Michan, C., Rojo, F., Dwyer, D. & Timmis, K. Signal-regulator interactions. Genetic analysis of the effector binding site of XylS, the benzoate-activated positive regulator of Pseudomonas TOL plasmid meta-cleavage pathway operon. J. Mol. Biol. 211, 373-82 (1990).
Ramos, J. L., Stolz, A., Reineke, W. & Timmis, K. N. Altered effector specificities in regulators of gene expression: TOL plasmid xylS mutants and their use to engineer expansion of the range of aromatics degraded by bacteria. Proc. Natl. Acad. Sci. USA 83, 8467-71 (1986).
Reetz, M. T. Combinatorial and evolution-based methods in the creation of enantioselective catalysts. Angew. Chem., Int. Ed. 40, 284-310 (2001).
Reetz, M. T. Screening for enantioselective enzymes. Enzyme Functionality, 559-598 (2004).
Robertson, D. E. et al. Exploring nitrilase sequence space for enantioselective catalysis. Appl. Environ. Microbiol. 70, 2429-2436 (2004).
Salto, R., Delgado, A., Michan, C., Marques, S. & Ramos, J. L. Modulation of the function of the signal receptor domain of XylR, a member of a family of prokaryotic enhancer-like positive regulators. J. Bacteriol. 180, 600-4 (1998).
Sambrook, J., & Russell, D. W. Molecular Cloning: A Laboratory Manual. New York, NY, USA. Cold Spring Harbor Laboratory Press (2001).
Samuelson, P., Gunneriusson, E., Nygren, P.-A. & Stahl, S. Display of proteins on bacteria. J. Biotechnol. 96, 129-154 (2002).
Schell, M. A., Brown, P. H. & Raju, S. Use of saturation mutagenesis to localize probable functional domains in the NahR protein, a LysR-type transcription activator. J. Biol. Chem. 265, 3844-50 (1990).
Schmid, A., Kollmer, A., Mathys, R. G. & Witholt, B. Developments toward largescale bacterial bioprocesses in the presence of bulk amounts of organic solvents. Extremophiles 2, 249-256 (1998).
Schnappinger, D. and W. Hillen. Tetracyclines: antibiotic action, uptake, and resistance mechanisms. Arch. Microbiol. 165 (6), 359-69 (1996).
Scholz, O., Kostner, M., Reich, M., Gastiger, S. & Hillen, W. Teaching TetR to Recognize a New Inducer. J. Mol. Biol. 329, 217-227 (2003).
Schumacher Maria, A. & Brennan Richard, G. Structural mechanisms of multidrug recognition and regulation by bacterial multidrug transcription factors. Mol. Microbiol. 45, 885-93. (2002).
Shingler, V. & Moore, T. Sensing of aromatic compounds by the DmpR transcriptional activator of phenol-catabolizing Pseudomonas sp. strain CF600. J. Bacteriol. 176, 1555-60 (1994).
Skarfstad, E., O'Neill, E., Garmendia, J. & Shingler, V. Identification of an effector specificity subregion within the aromatic-responsive regulators DmpR and XylR by DNA shuffling. J. Bacteriol. 182, 3008-16 (2000).
Smirnova, I. A. et al. Development of a bacterial biosensor for nitrotoluenes: the crystal structure of the transcriptional regulator DntR. J. Mol. Biol. 340, 405-18 (2004).
Smits, T. H. M., M. A. Seeger, et al. New Alkane-Responsive Expression Vectors for Escherichia coli and Pseudomonas. Plasmid 46 (1), 16-24 (2001).
Stover, C. K. et al. Complete genome sequence of Pseudomonas aeruginosa PA01. Nature 406, 959-964 (2000).
Studier, W. F., A. H. Rosenberg, et al. Use of T7 RNA polymerase to direct expression of cloned genes. Methods Enzymol. 185, 60-89 (1990).
Sutherland, J. D. Evolutionary optimisation of enzymes. Curr Opin Chem Biol 4, 263-9 (2000).
Tropel, D. & van der Meer, J. R. Bacterial transcriptional regulators for degradation pathways of aromatic compounds. Microbiol. Mol. Biol. Rev. 68, 474-500 (2004).
Uchiyama, T., Abe, T., Ikemura, T. & Watanabe, K. Substrate-induced gene-expression screening of environmental metagenome libraries for isolation of catabolic genes. Nat. Biotechnol. 23, 88-93 (2005).
Williams, J.M.J. Catalysis in Asymmetric Synthesis. Sheffield Academic Press (1999).
Wise, A. A. & Kuske, C. R. Generation of novel bacterial regulatory proteins that detect priority pollutant phenols. Appl. Environ. Microbiol. 66, 163-169 (2000).
Wright, G. D. Bacterial resistance to antibiotics: Enzymatic degradation and modification. Advanced Drug Delivery Reviews 57 (10), 1451-1470 (2005).
Yen, K. M. Construction of cloning cartridges for development of expression vectors in gram-negative bacteria. J Bacteriol. 173 (17), 5328-35 (1991).

## Claims

1. A method of detecting among a population of candidate biocatalysts a biocatalyst capable of catalyzing a chemical conversion reaction from a substrate to a product, said method comprising the steps of:
a) providing a host cell comprising:
- at least one product-inducible expression system comprising nucleic acid encoding at least one detector gene operably linked to a regulatory element, wherein the expression of said detector gene is inducible by said product, and
- at least one biocatalyst expression system comprising nucleic acid encoding at least one candidate biocatalyst wherein said at least one candidate biocatalyst is selected from said population of candidate biocatalysts,
b) contacting said host cell with said substrate under conditions wherein said nucleic acid encoding at least one candidate biocatalyst is expressed in said host cell and wherein said substrate is allowed to contact said candidate biocatalyst, and wherein said substrate is converted into said product in case said candidate biocatalyst is capable of catalyzing said reaction, and
c) detecting said host cell as one comprising a biocatalyst capable of catalyzing said chemical conversion reaction on the basis of the expression of said detector gene.

2. Method according to claim 1, wherein step a) comprises the provision of a multitude of host cells that represents said population of candidate biocatalysts, wherein said multitude of host cells:
- is a library of cells of a single cell type wherein essentially each host cell comprises a different cloned nucleic acid fragment encoding at least one candidate biocatalyst, or
- are cells of different cell types wherein essentially each host cell comprises a different candidate biocatalyst.

3. Method according to claim 1 or 2, wherein said host cell is a bacterial cell, a microbial cell, a plant cell, or an animal cell.

4. Method according to any one of claims 1-3, wherein the expression of said detector gene interferes with cell mobility, cell division, cell metabolism, cell density, cell surface molecules or product excretion.

5. Method according to any one of claims 1-4, wherein said detector gene is a reporter gene, preferably a gene for a fluorogenic or chromogenic marker.

6. Method according to any one of claims 1-4, wherein said detector gene is a gene for antibiotic resistance.

7. Method according to any one of the preceding claims, wherein said product-inducible expression of said detector gene is brought about by binding of said product to a regulatory protein and wherein said regulatory protein has the ability to bind to DNA and activate transcription of said at least one product-inducible expression system.

8. Method according to claim 7, wherein said regulatory element of said at least one product-inducible expression system is a binding site for said regulatory protein.

9. Method according to any one of the preceding claims, wherein said conditions under which said host cell is contacted with said substrate provide an inducer for activating the expression of said at least one biocatalyst expression system.

10. Method according to any one of the preceding claims, wherein said conditions under which said host cell is contacted with said substrate provide a growth medium comprising an antibiotic compound for which the resistance is encoded by said at least one detector gene.

11. Method according to any one of the preceding claims, wherein said at least one biocatalyst expression system comprises nucleic acid encoding at least two candidate biocatalysts each capable of catalyzing at least one reaction of a multi-step chemical conversion reaction.

12. Method according to claim 11, wherein the product of the reaction catalyzed by one of said at least two candidate biocatalysts is the substrate for the reaction catalyzed by another one of said at least two candidate biocatalysts.

13. Method according to claim 11 or 12, wherein said nucleic acid encoding said at least two candidate biocatalysts is comprised on separate expression systems for the separate candidate biocatalysts.

14. Method according to any one of the preceding claims, further comprising the step of d) providing said biocatalyst by selecting said host cell(s) detected in step c).

15. A host cell suitable for use in a method of detecting among a population of candidate biocatalysts a biocatalyst capable of catalyzing the reaction from a substrate to a product, said host cell comprising:
a) at least one product-inducible expression system comprising nucleic acid encoding at least one detector gene operably linked to a regulatory element, wherein the expression of said detector gene is inducible by said product, and
b) at least one biocatalyst expression system comprising nucleic acid encoding at least one candidate biocatalyst, wherein said at least one candidate biocatalyst is selected from said population of candidate biocatalysts.

16. Host cell according to claim 15, wherein said host cell is one cell out of a multitude of host cells that represents said population of candidate biocatalysts, wherein said multitude of host cells:
- is a library of cells of a single cell type wherein essentially each host cell comprises a different cloned nucleic acid fragment encoding at least one candidate biocatalyst, or
- are cells of different cell types wherein essentially each host cell comprises a different candidate biocatalyst.

17. Host cell according to claim 15 or 16, wherein said host cell is a bacterial cell, a microbial cell, a plant cell, or an animal cell.

18. Host cell according to any one of claims 15-17, wherein said detector gene is a gene for a chromogenic marker or a gene for a resistance marker.

19. Host cell according to any one of claims 15-18, wherein said product-inducible expression of said detector gene is brought about by binding of said product to a regulatory protein and wherein said regulatory protein has the ability to bind to DNA and activate transcription of said at least one product-inducible expression system.

20. Host cell according to claim 19, wherein said regulatory element of said at least one product-inducible expression system is a binding site for said regulatory protein.

21. Host cell according to any one of claims 15-20, wherein said biocatalyst expression system can be activated by an externally provided inducer.

22. Host cell according to any one of claims 15-21, wherein said at least one biocatalyst expression system comprises nucleic acid encoding at least two candidate biocatalysts each capable of catalyzing at least one reaction of a multi-step chemical conversion reaction.

23. Host cell according to claim 22, wherein said nucleic acid encoding said at least two candidate biocatalysts is comprised on separate expression systems for the separate candidate biocatalysts.

24. A method of selecting among a population of candidate substrates a substrate capable of being converted into a product in a biocatalyst-catalyzed chemical conversion reaction, said method comprising the steps of
a) providing a host cell according to any one of claims 15-23;
b) subjecting said host cell to conditions wherein said detector gene allows for the detection of cells capable of forming said product, and
c) selecting said substrate on the basis of the expression of said detector gene.

25. A method of producing a (bio)chemical compound wherein said compound is the product of a biocatalyst-catalyzed chemical conversion reaction for converting substrate into product, said method comprising the steps of:
a) providing at least one biocatalyst or set of biocatalysts by a method according to claim 14;
b) producing said at least one biocatalyst or set of biocatalysts in at least one production strain, or using the selected host cell(s) as a production strain(s);
c) providing a reaction mixture comprising said production strain(s) and at least one substrate and optional cofactors for the chemical conversion reaction catalyzed by said at least one biocatalyst or set of biocatalysts, and
d) allowing said substrate to be converted by said at least one biocatalyst or set of biocatalysts, thereby providing the (bio)chemical compound as the reaction product.
